# EUROPEAN PATENT APPLICATION

(11) **EP 4 393 394 A1**
(43) Date of publication of application: **03.07.2024**
(21) Application number: 21954810.4
(22) Date of filing: 25.11.2021
(51) Int. Cl.: A61B 5/145

(54) **APPLICATION APPARATUS FOR APPLYING MEDICAL INSTRUMENT TO HOST**

(30) Priority: 26.08.2021 CN 202110990054
(71) Applicant: Shenzhen Sisensing Technology Co., Ltd., Shenzhen, Guangdong 518000 (CN)
(72) Inventor: LI, Yunfeng, Shenzhen, Guangdong 518000 (CN); CHEN, Zhi, Shenzhen, Guangdong 518000 (CN); PENG, Weibin, Shenzhen, Guangdong 518000 (CN); GONG, Mingli, Shenzhen, Guangdong 518000 (CN); WU, Jiang, Shenzhen, Guangdong 518000 (CN); HUANG, Xiuliang, Shenzhen, Guangdong 518000 (CN); HAN, Mingsong, Shenzhen, Guangdong 518000 (CN)
(74) Representative: Isern Patentes y Marcas S.L.
(86) International application number: PCT/CN2021/133305
(87) International publication number: WO 2023/024293

(57) **Abstract**

The present disclosure describes an applicator for applying a medical instrument to a host, comprising a housing, an auxiliary mechanism, a first drive mechanism and a second drive mechanism, the auxiliary mechanism comprising a moving body movable with respect to the housing, a receiving portion provided to the moving body and configured to receive and accommodate the medical instrument, and a puncture member configured to be movable with respect to the receiving portion, the first drive mechanism being configured to act on the moving body toward a proximal end, the second drive mechanism being configured to act on the puncture member toward a distal end, when the moving body is released, the moving body being driven toward the proximal end by the first drive mechanism to place the medical instrument received in the receiving portion at least partially subcutaneously in the host through the puncture member; and the puncture member being driven toward the distal end by the second drive mechanism to move the puncture member away from the host. According to the present disclosure, it is able to provide an applicator which is easy to operate and can accurately apply the medical

## Description

### BACKGROUND OF INVENTION

### Field of Invention

The present disclosure relates to an applicator for applying a medical instrument to a host.

### Description of Related Art

For the needs of clinical diagnosis or personal health monitoring, for example, for diabetic patients, it is quite necessary to monitor the glucose concentration in interstitial fluid in real time and continuously on a regular basis, and to adjust the glucose concentration in time by, for example, adjusting diet or applying medication, so as to reduce and lower the occurrence of complications due to abnormal glucose concentration.

Currently, users typically monitor glucose concentration in interstitial fluid using a glucose sensor that can be inserted subcutaneously and can react with glucose in the interstitial fluid, and an electronic device that is attached to the skin and connected to the glucose sensor. In order to insert the glucose sensor subcutaneously and attach the electronic device to the skin, generally it is necessary to resort to an insertion device. Specifically, the insertion device may include a needle capable of piercing the skin, and insert the glucose sensor below skin through the needle, then separate the needle from the glucose sensor and remove it from the skin.

However, in the above-mentioned prior art, the process of piercing the needle into the skin or removing the needle out of the skin needs to be driven by means of human power. In this case, since it may be difficult to obtain more precise control of the force with which the needle is driven, the location at which the needle is inserted into the skin, the depth at which the needle is inserted below the skin, or the timing at which the needle is removed out of the skin, on the one hand, it may affect the user's experience when the glucose sensor is inserted, and on the other hand, it may result that the accuracy of the obtained glucose concentration is not high because the glucose sensor is not inserted into the desired location.

### SUMMARY OF INVENTION

The present invention has been made in diagram of the above-mentioned state of the art, and an object thereof is to provide an applicator which is easy to operate and can accurately apply a medical instrument to a host.

For the above purpose, the present invention provides an applicator for a medical instrument comprising a housing, an auxiliary mechanism, a first drive mechanism and a second drive mechanism, the housing comprising a first holding portion, a proximal end close to the host and a distal end remote from the host in operation, the auxiliary mechanism comprising a moving body releasably held by the first holding portion and configured to be relatively movable to the housing when released, a receiving portion provided to the moving body and configured to receive and accommodate the medical instrument, a second holding portion provided to the moving body, and a puncture member releasably held by the second holding portion and configured to be relatively movable to the receiving portion when released, the first drive mechanism being configured to act on the moving body in a manner toward the proximal end, the second drive mechanism being configured to act on the puncture member in a manner toward the distal end, when released, the moving body being driven by the first drive mechanism toward the proximal end to place the medical instrument received in the receiving portion at least partially subcutaneously in the host through the puncture member, and the puncture member being driven toward the distal end by the second drive mechanism to move the puncture member away from the host.

In the applicator according to the present embodiment, the housing includes a proximal end proximal to the host and a distal end distal from the host when in operation, a receiving portion is configured to receive and accommodate the medical instrument, and a puncture member is movable relative to the receiving portion and disposed in the moving body, the moving body is driven towards the proximal end by a first drive mechanism to at least partially place the medical instrument subcutaneously in the host, and the puncture member is driven towards the distal end by a second drive mechanism to move the puncture member away from the host. In this case, the two operating travels required during application of the medical instrument, i.e. placing the medical instrument subcutaneously in the host by means of the puncture member and moving the puncture member away from the host, are configured to be applied by the drive mechanism, whereby the medical instrument can be applied to the host easily. In addition, the housing comprises a proximal end which, in operation, is close to the host, by means of which it is possible to facilitate to define an application position of the medical instrument, and by virtue of the driving action precisely provided by the drive mechanism relatively, , the puncture force, the puncture depth, and the withdrawn timing from the host of the puncture member during application can be controlled more accurately, whereby it is also facilitated that the medical instrument can be applied to the host precisely.

Further, in the applicator according to the present embodiment, optionally, the moving body comprises a first locking portion configured to releasably interlock with the first holding portion. Thus, the moving body can be releasably held by the first holding portion through the engagement of the first locking portion and the first holding portion.

Further, in the applicator according to the present embodiment, optionally, the first holding portion and the first locking portion are releasably interlocked by at least one structure of a buckle, a hook, a latch, or a pin. In this case, the first holding portion and the first locking portion are releasably interlocked by at least one of a buckle, a hook, a latch, or a pin, thereby providing an interlock which is easy to be released.

In addition, the applicator according to the present embodiment optionally further comprises a first trigger mechanism configured to enable the first holding portion to separate from the first locking portion for releasing the moving body. Thereby, the moving body can be easily released via the first trigger mechanism.

Further, in the applicator according to the present embodiment, optionally, the puncture member comprises a second locking portion configured to releasably interlock with the second holding portion. Thus, the puncture member can be releasably held by the second holding portion through engagement of the second locking portion with the second holding portion.

Further, in the applicator according to the present embodiment, optionally, he second holding portion and the second locking portion are releasably interlocked by at least one structure of a buckle, a hook, a latch, or a pin. In this case, the second holding portion and the second locking portion are releasably interlocked by at least one of a buckle, a hook, a latch, or a pin, thereby providing an interlock which is easy to be released.

In addition, the applicator according to the present embodiment optionally further comprises a second trigger mechanism configured to enable the second holding portion to separate from the second locking portion for releasing the puncture member. Thereby, the puncture member can be released easily via the second trigger mechanism.

Further, in the applicator according to the present embodiment, optionally, the housing comprises a first housing having the first holding portion and a second housing mountable to the first housing, the second housing has a first limiting mechanism and a second limiting mechanism communicating with each other, the second limiting mechanism is configured to define an attachment position of the medical instrument, the auxiliary mechanism is mounted to the first limiting mechanism and movable along the first limiting mechanism when the moving body is released by the first holding portion. In this case, the movement of the moving body is defined by the first limiting mechanism of the second housing, and the attachment position of the medical instrument is defined by the second limiting mechanism of the second housing, whereby more accurate application of the medical instrument to the host can be facilitated.

Further, in the applicator according to the present embodiment, optionally, the moving body has a first bottom portion close to the distal end, a second bottom portion close to the proximal end, a side wall connecting the first bottom portion and the second bottom portion, and a hollow portion formed between the first bottom portion, the second bottom portion, and the side wall, the receiving portion being provided at the second bottom portion and movable along the hollow portion when the puncture member is released. In this case, the hollow portion is formed with the first bottom portion, the second bottom portion, and the side wall, and the receiving portion is provided to the second bottom portion, and the puncture member is provided to the hollow portion, whereby the puncture member's movement relative to the receiving portion can be facilitated when the puncture member is released.

Further, in the applicator according to the present embodiment, optionally, the puncture member comprises a sharp object having a groove, and a bearing for supporting the sharp object, the medical instrument may be wholly or partially placed in the groove of the sharp object, and the second drive mechanism is configured to apply an action on the bearing. In this case, by placing the medical instrument wholly or partly in the groove of the sharp object, it is thereby able to facilitate to place the medical instrument at least partly subcutaneously in the host by means of the puncture member.

Further, in the applicator according to the present embodiment, optionally, the medical instrument comprises a sensor that can be placed subcutaneously in the host, and an attachment portion that is connected to the sensor and can be attached to a body surface of the host. In this case, it is able to facilitate the acquisition of the analyte information of the tissue fluid through the sensor that can be placed subcutaneously in the host and connected to the sensor with the attachment portion that can be attached to a body surface of the host.

Further, in the applicator according to the present embodiment, optionally, the first holding portion has a surface facing the distal end, and the first locking portion has a surface facing the proximal end, the surface facing the distal end of the first holding portion is engaged with the surface facing the proximal end of the first locking portion when the moving body is held, and the surface facing the distal end of the first holding portion is separated from the surface facing the proximal end of the first locking portion when the moving body is released.

Further, in the applicator according to the present embodiment, optionally, at least a portion of the first holding portion is "L"-shaped or hook-shaped. Thus, it is facilitated to easily form a buckle or the like to interlock with the first locking portion.

Further, in the applicator according to the present embodiment, optionally, the first holding portion has an arm extending substantially in a direction of a central axis of the applicator, and a protrusion cooperating with the arm and projecting substantially in a direction orthogonal to the central axis of the applicator. In this case, when the protrusion of the first holding portion overlaps the first locking portion, the protrusion is actuated by the actuating arm, whereby the first locking portion can be easily released by the first holding portion.

Further, in the applicator according to the present embodiment, optionally, the arm of the first holding portion is resilient in the direction substantially orthogonal to the central axis of the applicator.

Further, in the applicator according to the present embodiment, optionally, the arm of the first holding portion converges toward the central axis of the applicator in a direction from the distal end to the proximal end, and the protrusion of the first holding portion projects toward the central axis of the applicator. In this case, the first locking portion can be held on the inner side (close to the central axis of the applicator) through gathering the first holding portion inward as a whole, and the first locking portion can be released through actuating the first holding portion toward the outer side (away from the central axis of the applicator), whereby the holding and releasing of the first locking portion can be facilitated.

Further, in the applicator according to the present embodiment, optionally, the arm of the first holding portion back-faces away from the central axis of the applicator in a direction from the distal end to the proximal end, and the protrusion of the first holding portion back-faces and projects away from the central axis of the applicator. In this case, the first locking portion can be held at the outer side (away from the central axis of the applicator) through dispersing the first holding portion outward as a whole, and the first locking portion can be released through actuating the first holding portion toward the inner side (close to the central axis of the applicator), whereby holding and releasing of the first locking portion can be facilitated.

Further, in the applicator according to the present embodiment, optionally, the first locking portion is substantially parallel to or away from a central axis of the applicator in a direction from the proximal end to the distal end. Thus, it is possible to facilitate the cooperation with the first holding portion gathered together inwardly to interlock.

Further, in the applicator according to the present embodiment, optionally, t the first locking portion is substantially parallel to or converges toward a central axis of the applicator in a direction from the proximal end to the distal end. Thereby, it is possible to facilitate cooperation with the outwardly dispersed first holding portion to interlock.

Further, in the applicator according to the present embodiment, optionally, the first trigger mechanism has an actuation portion extending substantially in the direction of the central axis of the applicator, and is configured to be movable in the direction of the central axis of the applicator. In this case, by driving the actuation portion of the first trigger mechanism in the central axis of the applicator, the first holding portion can be operated easily and actuated to be separated from the first locking portion.

Further, in the applicator according to the present embodiment, optionally, when projected in the direction of the central axis of the applicator, the projection of the actuation portion of the first trigger portion at least partially coincides with the projection of the first holding portion, and the first holding portion is actuated to move away from the first locking portion when the first trigger portion moves in the direction of the central axis of the applicator. Thus, the first locking portion can be released from the first holding portion with easy operation.

Further, in the applicator according to the present embodiment, optionally, when projected in the direction of the central axis of the applicator, the projection of the actuation portion of the first trigger portion at least partially coincides with the projection of the first locking portion, and the first locking portion is actuated to move away from the first holding portion as the first trigger portion moves in the direction of the central axis of the applicator. Thus, the first locking portion can be released with easy operation.

Further, in the applicator according to the present embodiment, optionally, the first housing comprises a hollow cylindrical peripheral portion and an end portion provided at one end of the peripheral portion near the distal end, and the first holding portion is provided at the end portion.

Further, in the applicator according to the present embodiment, optionally, one or plurality of rib shape protrusions are provided on an inner wall of the first housing, and one or plurality of rib shape grooves are provided on an outer wall of the second housing, and the one or plurality rib shape protrusions are respectively embedded into the one or plurality rib shape grooves when the second housing is mounted to the first housing. In this case, the assembling of the first housing with the second housing can be facilitated by the matching of the rib shape protrusions with the rib shape grooves.

Further, in the applicator according to the present embodiment, optionally, the first limiting mechanism and the second limiting mechanism are in hollow cylindrical shape and an inner diameter of the first limiting mechanism is not greater than an inner diameter of the second limiting mechanism. In this case, by providing the first limiting mechanism and the second limiting mechanism in a hollow cylindrical shape, it is possible to facilitate the movement of the auxiliary mechanism along the first limiting mechanism and the second limiting mechanism.

Further, in the applicator according to the present embodiment, optionally, when the moving body is released, the moving body is movable along the first limiting mechanism and the receiving portion is movable along the second limiting mechanism. In this case, by causing the moving body to move along the first limiting mechanism and the receiving portion to move along the second limiting mechanism, it is possible to apply the medical instrument accomodated in the receiving portion to a desired position of the body surface of the host more accurately.

Further, in the applicator according to the present embodiment, optionally, the first limiting mechanism limits a travel of the moving body in a direction of a central axis of the applicator. In this case, the travel of the moving body in the direction along the central axis of the applicator is limited by the first limiting mechanism, whereby the medical instrument accommodated in the receiving portion can be pushed a predetermined distance more accurately, so that the medical instrument can be applied to the host more accurately.

Further, in the applicator according to the present embodiment, optionally, when projected in the direction of the central axis of the applicator, a projection of the receiving portion coincides at least partially with a projection of a wall of the first limiting mechanism, and a projection of the end, near the distal end, of the moving body coincides at least partially with a projection of the wall of the first limiting mechanism. In this case, by making the projection of the receiving portion and the projection of the moving body at least partially coincide with the projection of the wall of the first limiting mechanism, it is thereby possible to effectively limit the travel of the moving body moving along the first limiting mechanism with simplified structure.

Further, in the applicator according to the present embodiment, optionally, the first limiting mechanism inhibits a rotation of the moving body. In this case, by inhibiting the rotation of the moving body, undesired rotation of the puncture member during application can thereby be inhibited.

Further, in the applicator according to the present embodiment, optionally, the first limiting mechanism comprises a groove (grooves) and/or a ridge(ridges) provided continuously or discontinuously on an inner wall substantially in a direction of the central axis of the applicator. In this case, by engaging the moving body with the groove(s) and/or ridge(s), undesired rotation of the moving body within the first limiting mechanism can be effectively inhibited.

Further, in the applicator according to the present embodiment, optionally, a protrusion back-facing away from a central axis of the applicator in a direction generally orthogonal to the central axis of the applicator is provided on an end, near the distal end, of the moving body. In this case, over-travel of the moving body toward the proximal end can be effectively restricted by the buckle formed by the protrusion and the wall of the first limiting mechanism.

Further, in the applicator according to the present embodiment, optionally, the moving body comprises a groove (grooves) and/or a ridge (ridges) provided on an outer wall of a side wall substantially in a direction of a central axis of the applicator. In this case, by engaging the groove(s) and/or ridge(s) of the moving body with the groove(s) and/or ridge(s) of the first limiting mechanism, undesired rotation of the moving body within the first limiting mechanism can be effectively inhibited.

Further, in the applicator according to the present embodiment, optionally, the moving body has a cut substantially in a direction of a central axis of the applicator. In this case, by providing a cut in the moving body in the direction of the central axis of the applicator, it is thereby possible to facilitate the application of the puncture member provided in the hollow portion of the moving body.

Further, in the applicator according to the present embodiment, optionally, a protrusion protruding toward the central axis of the applicator via the cut, is provided on an inner wall of the first limiting mechanism. In this case, when the moving body moves along the first limiting mechanism, the protrusion provided on the inner wall of the first limiting mechanism can act on the puncture member via the cut of the moving body, whereby a trigger mechanism for the puncture member can be provided with a simplified structure.

Further, in the applicator according to the present embodiment, optionally, the moving body is configured to inhibit a rotation of the puncture member. In this case, the user experience during application of the medical instrument can be improved by inhibiting undesired rotation of the puncture member during application.

Further, in the applicator according to the present embodiment, optionally, the moving body comprises a groove (grooves) and/or a ridge (ridges) provided on an inner wall of a side wall substantially in a direction of a central axis of the applicator. In this case, undesired rotation of the puncture member during application can be effectively inhibited by the groove(s) and/or ridge(s) provided on the inner wall of the side wall of the moving body cooperating with the groove(s) and/or ridge(s) of the puncture member.

Further, in the applicator according to the present embodiment, optionally, the sharp object is integrally formed with the bearing.

Further, in the applicator according to the present embodiment, optionally, the sharp object is configured for removable engagement with the bearing. Thus, additional sterilization of the sharp object can be facilitated.

Further, in the applicator according to the present embodiment, optionally, the sharp object has a needle shape portion and a cover portion in fixed connection with the needle shape portion, and the bearing has a plurality of finger-shape portions converging toward one another, and the cover portion is releasably clamped by the plurality of finger-shape portions. In this case, assembling and disassembling between the sharp object and the bearing can be facilitated by the cooperation of the cover of the sharp object and the plurality of finger-shape portions of the bearing.

Further, in the applicator according to the present embodiment, optionally, the puncture member comprises a second locking portion configured to releasably interlock with the second holding portion and provided on the bearing, the second locking portion comprises an arm extending substantially in a direction of a central axis of the applicator, and a protrusion cooperating with the arm and back-facing away from the central axis of the applicator substantially in a direction orthogonal to the central axis of the applicator. In this case, when the puncture member is placed within the hollow portion of the moving body, the second locking portion overlaps the cut of the moving body, whereby the puncture member can be held with a simplified structure.

Further, in the applicator according to the present embodiment, optionally, the arm of the second locking portion is resilient in the direction substantially orthogonal to the central axis of the applicator.

Further, in the applicator according to the present embodiment, optionally, the moving body has a cut substantially in the direction of the central axis of the applicator, and the protrusion of the second locking portion passes through the cut when the puncture member is held. In this case, the protrusion of the first locking portion overlaps the cut of the moving body, whereby the first locking portion can be held with a simplified structure.

Further, in the applicator according to the present embodiment, optionally, when the puncture member is released, the arm of the second locking portion is pressed toward the central axis of the applicator. Thus, the second locking portion can be released with easy operation.

Further, in the applicator according to the present embodiment, optionally, the bearing comprises a groove (grooves) and/or a ridge(ridges) provided on an outer wall substantially in the direction of the central axis of the applicator. In this case, undesired rotation of the puncture member can be effectively inhibited by the engagement of the groove(s) and/or ridge(s) of the bearing with the groove(s) and/or ridge(s) on the inner wall of the side wall of the moving body.

Further, in the applicator according to the present embodiment, optionally, the bearing of the puncture member is provided in the hollow portion, the receiving portion is provided at the second bottom portion, the second bottom portion has a through hole, and the sharp object passes through the through hole of the second bottom portion. In this case, the sharp object can be in engagement with the medical instrument accommodated in the receiving portion by arranging the sharp object to pass through the through hole of the second bottom portion, whereby it is able to facilitate at least partial placement of the medical instrument subcutaneously in the host by means of the puncture member.

Further, in the applicator according to the present embodiment, optionally, when the puncture member is held, a distance between the bearing and the first bottom portion is not less than a length of the sharp object protruding from the receiving portion. In this case, it is able to provide a space for movement of the puncture member away from the host by arranging the distance between the bearing and the first bottom portion to be not less than the length of the sharp object protruding from the receiving portion, whereby undesired damage of the puncture member to the host can be reduced.

Further, in the applicator according to the present embodiment, optionally, the second holding portion is releasably interlocked with the second locking portion by at least one of a buckle, a hook, a latch, or a pin when the puncture member is held. In this case, the second holding portion and the second locking portion are releasably interlocked by a simplified structure, whereby the second locking portion can be easily released.

Further, in the applicator according to the present embodiment, optionally, the second drive mechanism is disposed between the bearing and the second bottom portion. Thereby, it can be facilitated that the second drive mechanism acts on the puncture member away from the receiving portion.

Further, in the applicator according to the present embodiment, optionally, the second drive mechanism is a spring. Thereby, the actuation mechanism for the puncture member can be provided by a simplified structural design.

Further, in the applicator according to the present embodiment, optionally, the first drive mechanism is a spring. Thereby, it is able to provide the actuation mechanism for the moving body with a simplified structural design.

Further, in the applicator according to the present embodiment, optionally, the sensor is configured to react with an analyte in body fluid and generate analyte information. In this case, the sensor reacts with the analyte in the body fluid, whereby the acquisition of the analyte information in the body fluid can be facilitated.

Further, in the applicator according to the present embodiment, optionally, the analyte is one of or more than one of : acetylcholine, amylase, bilirubin, cholesterol, chorionic gonadotropin, creatine kinase, creatine, creatinine, DNA, fructosamine, glucose, glutamine, growth hormone, hormone, ketone body, lactate, oxygen, peroxide, prostate specific antigen, prothrombin, RNA, thyroid stimulating hormone, or troponin.

Further, in the applicator according to the present embodiment, optionally, the sensor comprises an implant portion which can be positioned subcutaneously in the host, and a connection portion connecting the implant portion to the attachment portion. In this case, it is able to facilitate transmission of the acquired analyte information to the external device by placing the implant portion subcutaneously in the host and connecting the implant portion to the attachment portion through the connection portion.

Further, in the applicator according to the present embodiment, optionally, the sensor is detachably mounted to the attachment portion. In this case, the sensor and the attachment portion may be packaged separately before the medical instrument is received in the receiving portion, thereby it is able to facilitate the sensor and the attachment portion to be sterilized separately.

Further, in the applicator according to the present embodiment, optionally, the attachment portion is configured to supply power to the sensor and receive information generated by the sensor. Thus, continuous monitoring of the sensor subcutaneously in the host can be facilitated.

Further, in the applicator according to the present embodiment, optionally, the attachment portion has a hole penetrating from an upper surface to a lower surface, and an axis of the sensor passes through the hole when the sensor is mounted to the attachment portion. In this case, the puncture member can be punctured into the skin subcutaneously through the hole, thereby it is able to facilitate to position the sensor subcutaneously.

Further, in the applicator according to the present embodiment, optionally, the attachment portion is configured to adhere to a body surface of the host.

Further, in the applicator according to the present embodiment, optionally, the attachment portion has an initial state configured to be an open circuit and an operation state configured to be a closed circuit. In this case, the attachment portion is configured to be open circuit when in the initial state, whereby power consumption can be saved before the attachment portion enters the operation state.

Further, in the applicator according to the present embodiment, optionally, the attachment portion is configured to communicate with an external device through wireless communication or wired communication. In this case, the acquired analyte information can be read in real time or periodically by configuring the attachment portion to communicate with an external device.

Further, in the applicator according to the present embodiment, optionally, the attachment portion is configured to be excited from the initial state to the operation state by magnetic action or light action. In this case, by exciting the attachment portion from the initial state to the operation state by a non-contact member such as a magnetic action or a light action, it is able to facilitate miniaturization of the attachment portion.

According to the present disclosure, it is able to provide an applicator which is easy to operate and can accurately apply a medical instrument to a host.

### BRIEF DESCRIPTION OF THE DRAWINGS

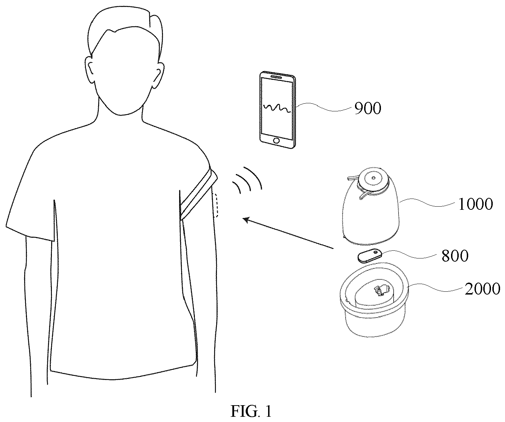

FIG. 1 is an application overview showing a medical instrument and an applicator according to an example of the present embodiment.

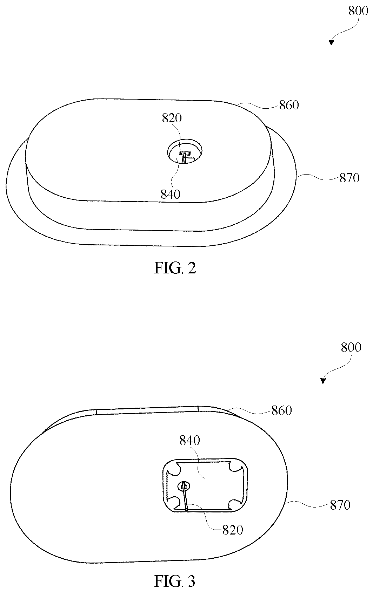

FIG. 2 is a schematic diagram showing a first perspective of a medical instrument according to an example of the present embodiment.
FIG. 3 is a schematic diagram showing a second perspective of a medical instrument according to an example of the present embodiment.

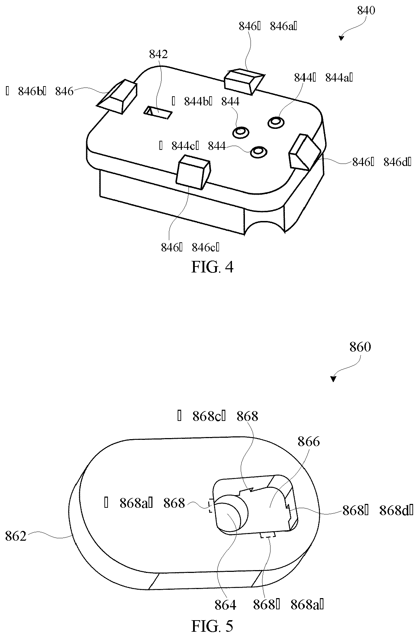

FIG. 4 is a schematic diagram showing a connection portion of a medical instrument according to an example of the present embodiment.
FIG. 5 is a schematic diagram showing an attachment portion of the medical instrument according to an example of the present embodiment.

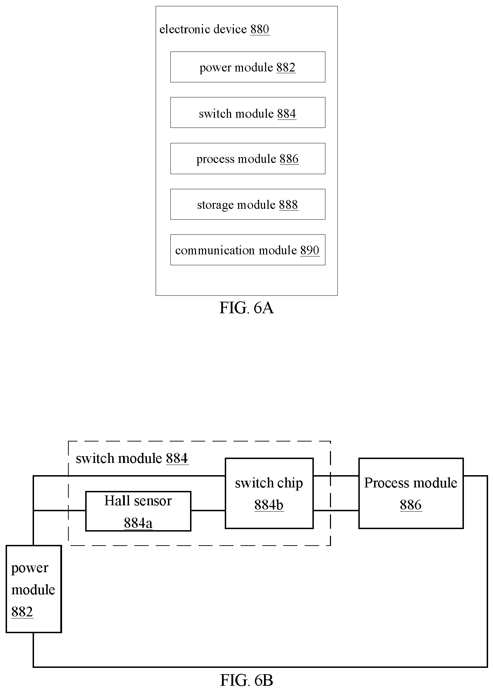

FIG. 6A is a schematic block diagram showing an electronic device of a medical instrument according to an example of the present embodiment. FIG. 6B is a schematic circuit block diagram showing an electronic device of a medical instrument according to an example of the present embodiment.

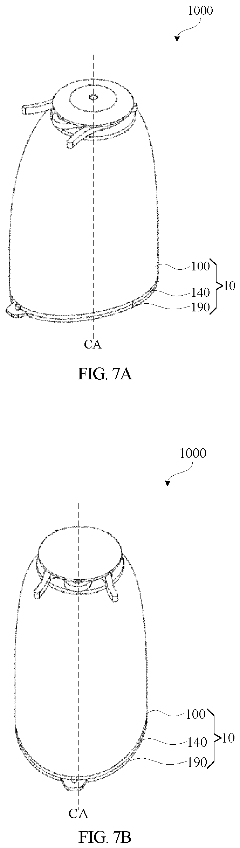

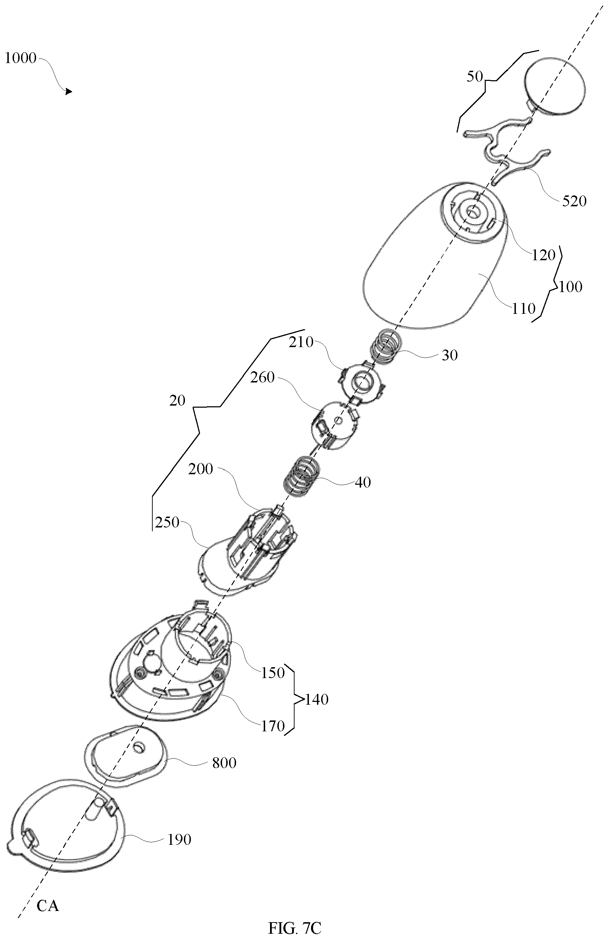

FIG. 7A is a schematic diagram showing an overall appearance of a first perspective of a applicator according to an example of the present embodiment FIG. 7B is a schematic diagram showing an overall appearance of a second perspective of a applicator according to an example of the present embodiment FIG. 7C is an exploded schematic diagram showing a applicator according to an example of the present embodiment

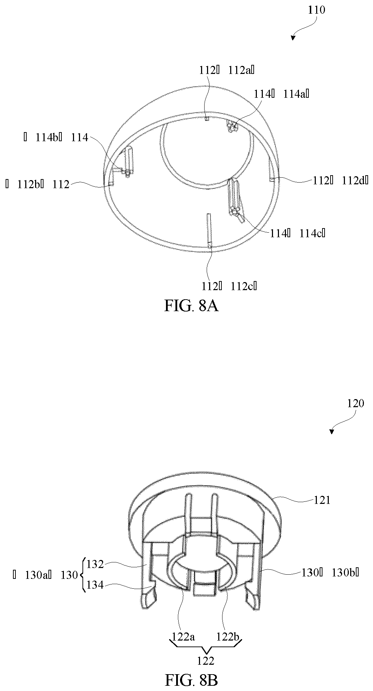

FIG. 8A is a schematic perspective diagram showing a peripheral portion of a first housing according to an example of the present embodiment. FIG. 8B is a schematic perspective diagram showing a first perspective of an end portion of the first housing according to an example of the present embodiment

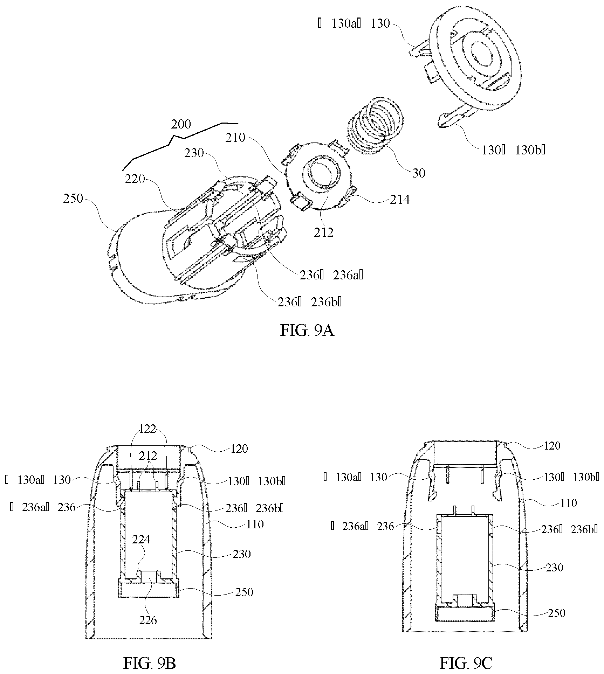

FIG. 9A is an exploded schematic diagram showing a first holding portion, a moving body, and a first drive mechanism according to an example of the present embodiment FIG. 9B is a schematic cross-sectional diagram showing a being-held state of a moving body according to an example of the present embodiment FIG. 9C is a schematic cross-sectional diagram showing a being-released state of a moving body according to an example of the present embodiment

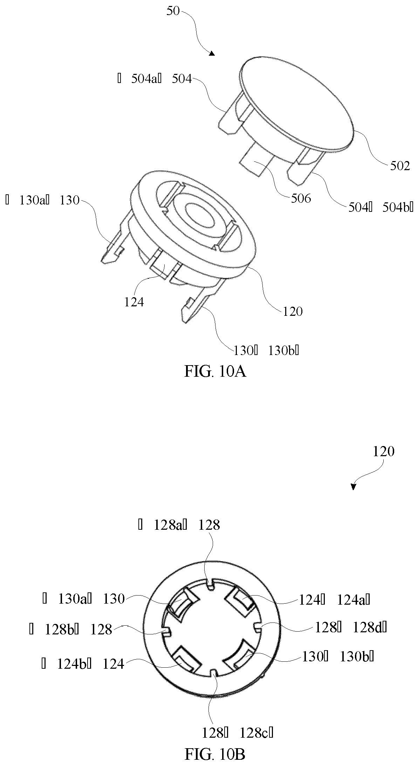

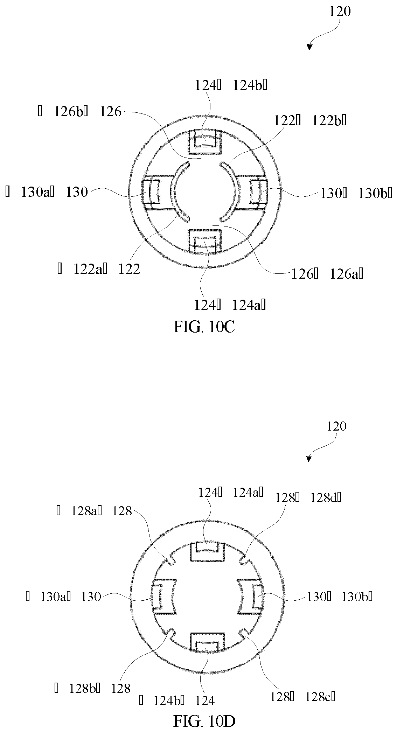

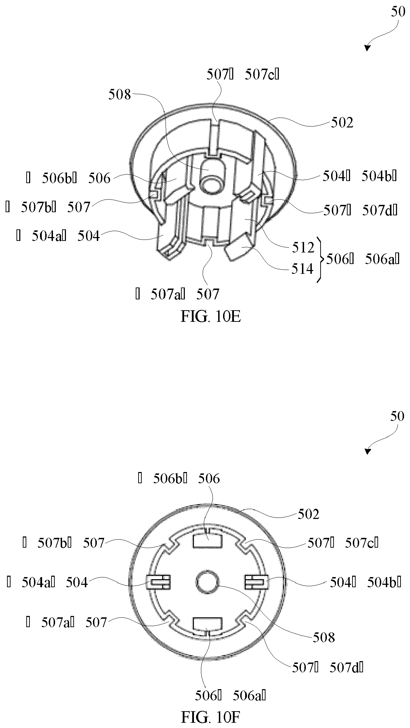

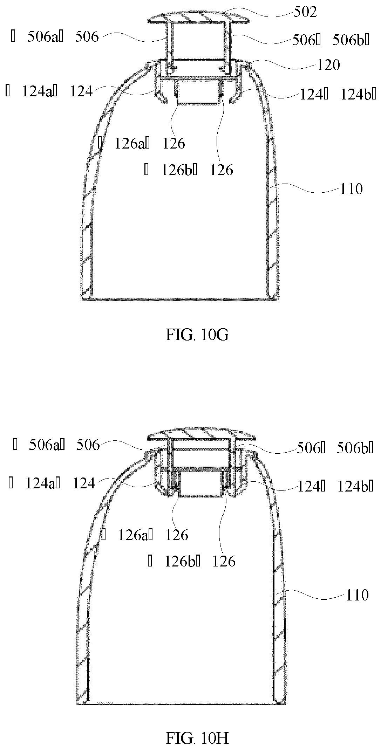

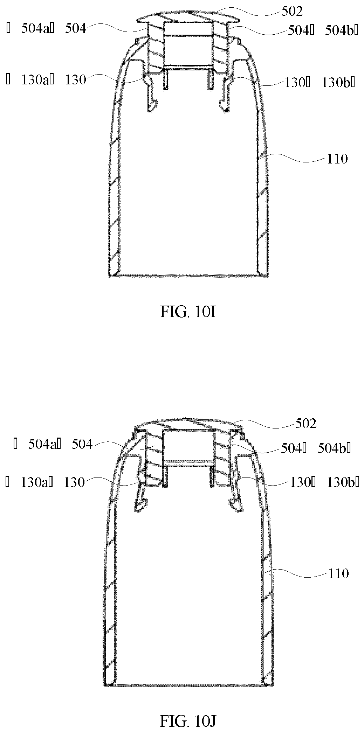

FIG. 10A is an exploded schematic diagram showing an end portion of a first housing and a first trigger mechanism according to an example of the present embodiment FIG. 10B is a schematic perspective diagram showing a second perspective of an end portion of a first housing according to an example of the present embodiment FIG. 10C is a schematic bottom diagram showing an end portion of a first housing according to an example of the present embodiment FIG. 10D is a schematic top diagram showing an end portion of a first housing according to an example of the present embodiment. FIG. 10E is a schematic perspective diagram showing a first trigger mechanism according to an example of the present embodiment FIG. 10F is a schematic bottom diagram showing a first trigger mechanism according to an example of the present embodiment FIG. 10G is a schematic cross-sectional diagram showing a first trigger mechanism according to an example of the present embodiment before the first trigger mechanism is mounted to an end portion. FIG. 10H is a schematic sectional diagram showing a first trigger mechanism according to an example of the present embodiment after the first trigger mechanism is mounted to an end portion. FIG. 10I is a schematic cross-sectional diagram showing a first holding portion according to an example of the present embodiment before triggered by a first trigger mechanism FIG. 10J is a schematic cross-sectional diagram showing a first holding portion according to an example of the present embodiment after triggered by a first trigger mechanism.

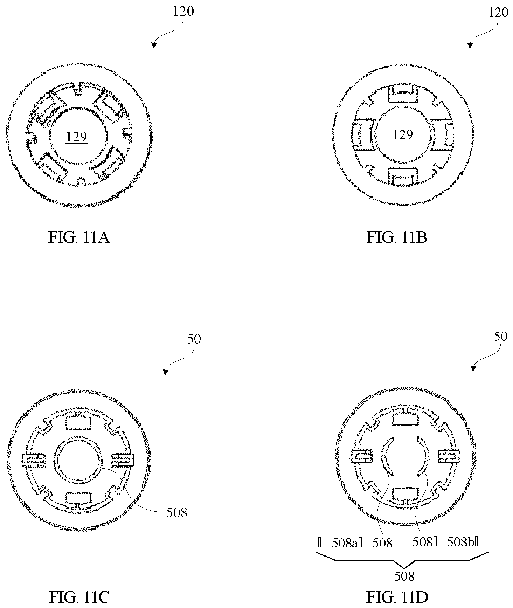

FIG. 11A is a schematic perspective diagram showing a second embodiment of an end portion according to an example of the present embodiment FIG. 11B is a schematic top diagram showing a second embodiment of an end portion according to an example of the present embodiment. FIG. 11C is a schematic bottom diagram showing a second embodiment of a first trigger mechanism according to an example of the present embodiment. FIG. 11D is a schematic bottom diagram showing a third embodiment of a first trigger mechanism according to an example of the present embodiment.

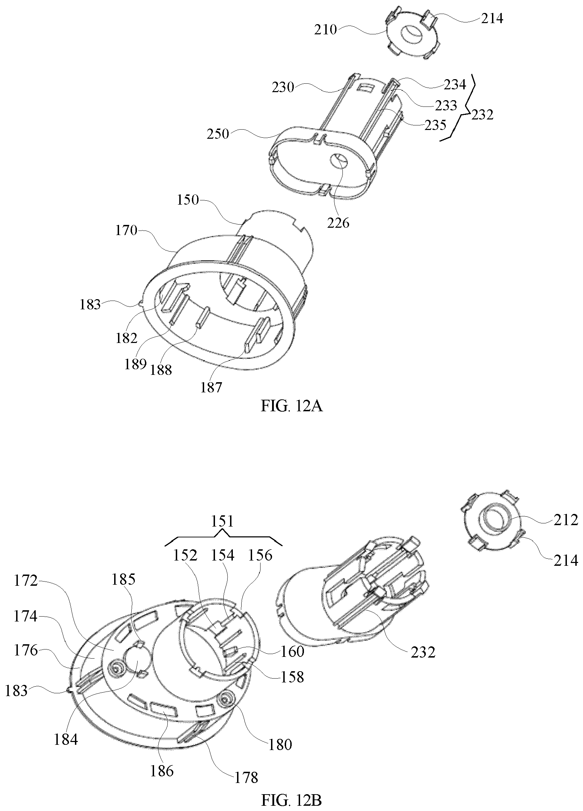

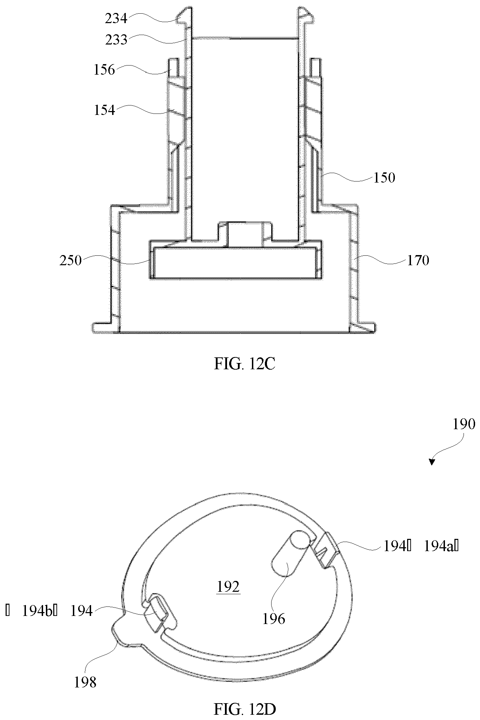

FIG. 12A is an exploded schematic diagram showing a first perspective of a second housing and an auxiliary mechanism. FIG. 12B is an exploded schematic diagram showing a second perspective of a second housing and an auxiliary mechanism. FIG. 12C is a cross-sectional schematic diagram showing an auxiliary mechanism mounted to a second housing. FIG. 12D is a schematic diagram showing a cover according to an example of the present embodiment.

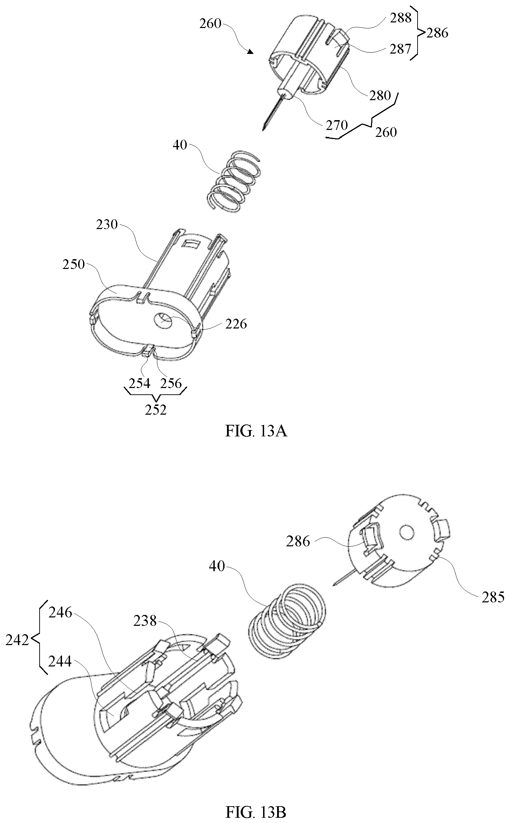

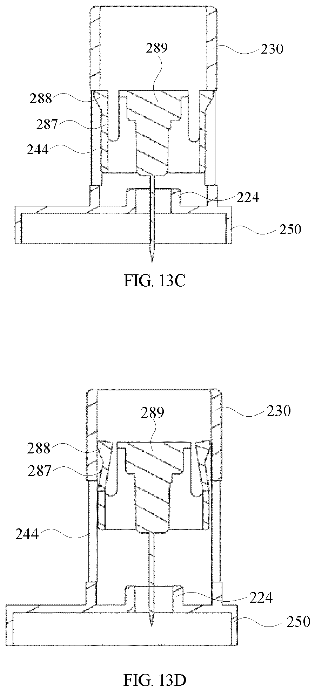

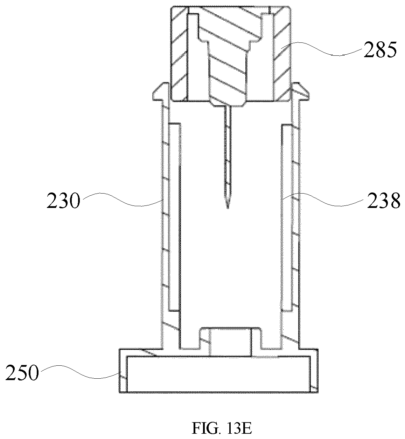

FIG. 13A is an exploded schematic diagram showing a first perspective of a moving body and apuncture member according to an example of the present embodiment FIG. 13B is an exploded schematic diagram showing a second perspective of a moving body and a puncture member according to an example of the present embodiment. FIG. 13C is a schematic cross-sectional diagram showing a state of being held of a puncture member according to an example of the present embodiment. FIG. 13D is a schematic cross-sectional diagram showing a state of being released of a puncture member according to an example of the present embodiment FIG. 13E is a schematic cross-sectional diagram illustrating pre-assembling of a moving body and a puncture member according to an example of the present embodiment.

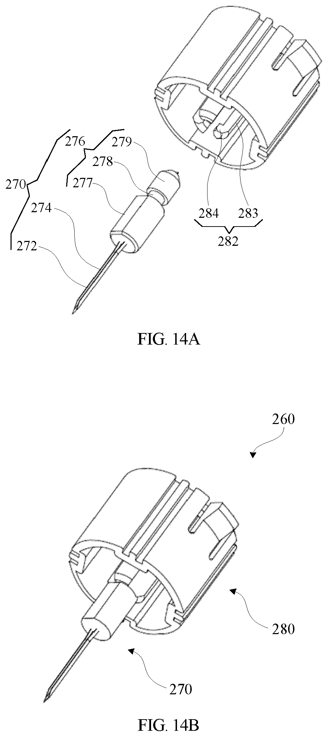

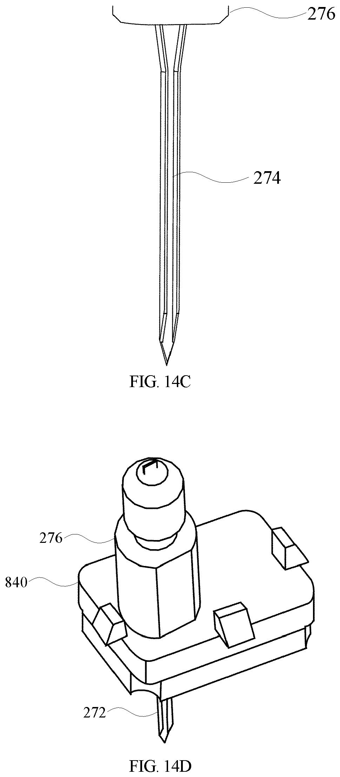

FIG. 14A is an exploded schematic diagram showing a puncture member according to an example of the present embodiment. FIG. 14B is a assembling schematic diagram showing a puncture member according to an example of the present embodiment FIG. 14C is an enlarged schematic diagram showing a needle shape portion according to an example of the present embodiment. FIG. 14D is a schematic diagram showing an assembling of a sharp object and a sensor according to an example of the present embodiment.

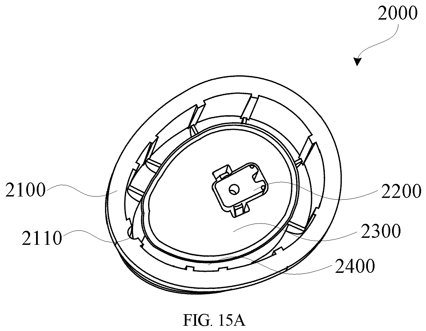

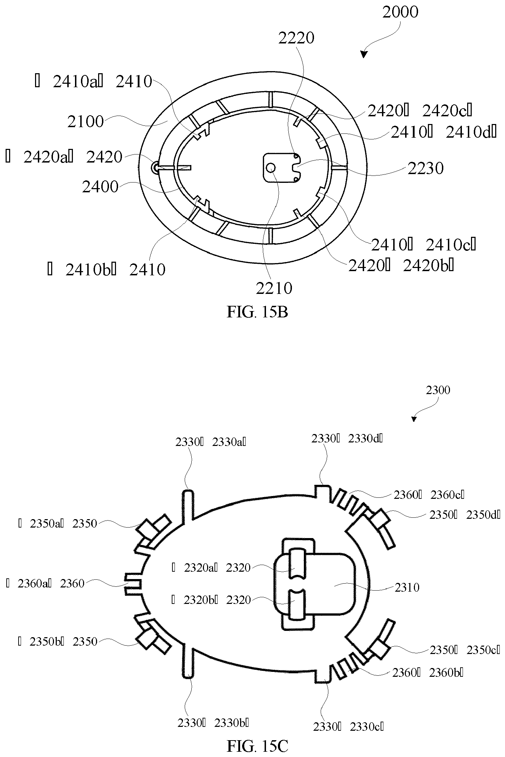

FIG. 15A is a schematic perspective diagram showing a cartridge device according to an example of the present embodiment. FIG. 15B is a schematic plan diagram showing a structure of a cartridge body, a loading table, and a fence structure of a cartridge device according to an example of the present embodiment FIG. 15C is a schematic top diagram showing a platform of a cartridge device according to an example of the present embodiment.

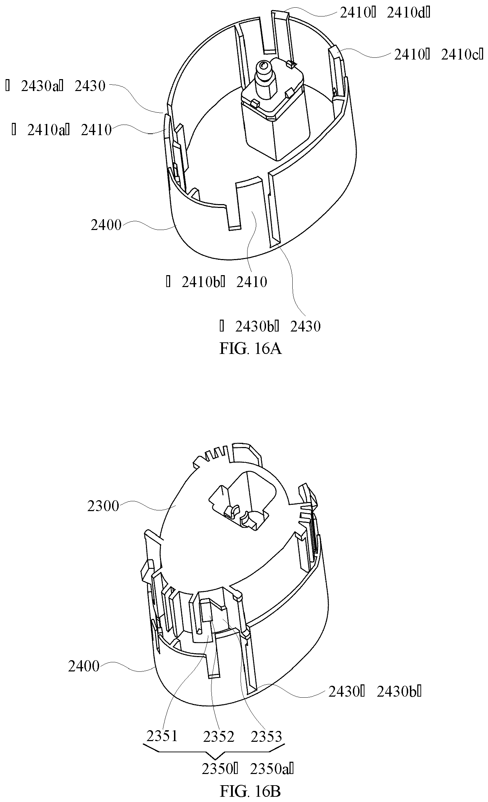

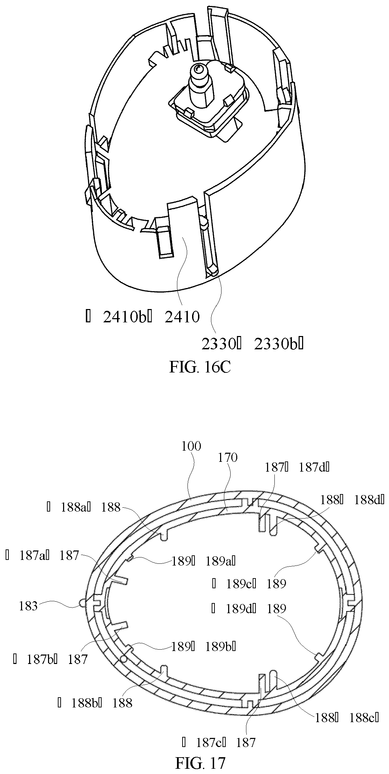

FIG. 16A is a schematic diagram showing a loading table onwhich a sensor is loaded according to an example of the present embodiment FIG. 16B is a schematic diagram showing that a platform according to an example of the present embodiment is higœr thana loading table. FIG. 16C is a schematic diagram showing that a platform according to an example of the present embodiment moves toward a bottom of a cartridge body so as to be lower than a loading table.
FIG. 17 is a sectional diagram showing the housing according to an example of the present embodiment in a direction substantially orthogonal to a central axis.

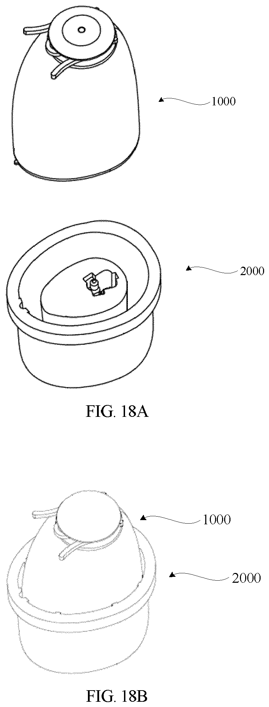

FIG. 18A is a schematic diagram showing a applicator and a cartridge device prior to their combination according to an example of the present embodiment. FIG. 18B is a schematic diagram showing aapplicatorand a cartridge device after their combination according to an example of the present embodiment.

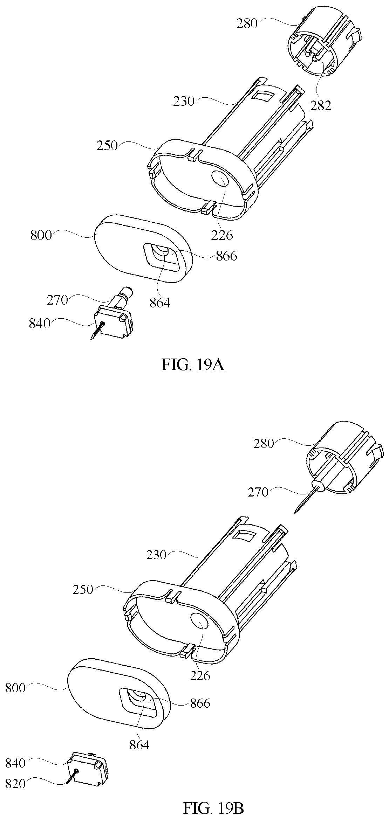

FIG. 19A is a schematic diagram showing a cartridge device storing a sensor in a first exemplary manner when the sensor is collected according to an example of the present embodiment. FIG. 19B is a schematic diagram showing a cartridge device storing a sensor in a second exemplary manner when the sensor is collected according to an example of the present embodiment

### DETAILED DESCRIPTION

Hereinafter; preferred embodiments of the present invention will be described in detail with reference to the accompanying drawings. In the following description, the same reference labels are given to the same components, and repeated descriptions are omitted. In addition, the drawings are merely schematic, and the proportions of the dimensions of the parts relative to each other; or the shapes of the parts, etc. may differ from the reality.

It should be noted that the terms "comprising" and "having", and any variations thereof, in this disclosure, such as a process, method, system, product, or apparatus that comprises or has a list of steps or elements are not necessarily restricted to those explicitly recited steps or elements, but may include or have other steps or elements not explicitly recited or inherent to such process, method, product, or apparatus.

Embodiments of the present disclosure relate to an applicator for a medical instrument that can be used to apply the medical instrument to a host, such as applying the medical instrument to a body surface of the host, or placing the medical instrument wholly or partially subcutaneously in the host, etc. With the applicator according to the present embodiment, it is able to facilitate the application of the medical instrument to a desired location.

The applicator according to the present embodiment may also be referred to as, for example, a boosting device, a transporting device, an implanting device, an attachment device, an auxiliary device, etc. It should be noted that various names are intended to indicate a device for applying the medical instrument to the host to which the present embodiment relates, and should not be understood as limitation or restriction

FIG. 1 is an application overview showing a medical instrument 800 and an applicator 1000 according to an example of the present embodiment. In the present embodiment, the medical instrument 800 can be applied to a host via the applicator 1000 and applied to a desired location, and the host can acquire physiological information through the medical instrument 800 applied to itself.

In addition, the present disclosure also provides a monitoring system that may include a medical instrument 800 related to the present embodiment that can acquire physiological information of a host, and a reading device 900 that can be communicatively connected to the medical instrument 800 (see FIG. 1). The medical instrument 800 applied to the host may transmit the acquired physiological information to the reading device 900, for example, wirelessly, thereby facilitating the host to read and monitor its own physiological information

In addition, the present disclosure also provides a cartridge device 2000 (see FIG. 1) that may be used to receive, in whole or in part, the medical instrument 800 according to the present embodiment. In some examples, the medical instrument 800 according to the present embodiment may be fully or partially received within the cartridge device 2000 prior to being applied to the host.

In addition, the present disclosure provides an instrument suite that may include the medical instrument 800 according to the present embodiment that can acquire physiological information of a host, the applicator 1000 according to the present embodiment that can apply the medical instrument 800 to the host, and the cartridge device 2000 according to the present embodiment that can receive the medical instrument 800. The medical instrument 800 may be wholly or partially received within the cartridge device 2000 before being applied to the host, and may be collected and applied to the host by the applicator 1000 when the medical instrument 800 is needed.

In some examples, the medical instrument 800 may be a sensing device. In some examples, medical instrument 800 may be an analyte sensor device that may generate information oft a particular analyte in body fluid based on the body fluid, etc., e.g. reacts with the analyte in the body fluid and generate analyte information. In this case, the sensor reacts with the analyte in the body fluid, whereby the acquisition of the analyte information in the body fluid can be facilitated.

In the present embodiment, the analyte targeted by the analyte sensor device may be one or more of glucose, acetylcholine, amylase, bilirubin, cholesterol, chorionic gonadotropin, creatine kinase, creatine, creatinine, DNA, fructosamine, glutamine, growth hormone, hormone, ketone body, lactate, oxygen, peroxide, prostate specific antigen, prothrombin, RNA, thyroid stimulating hormone, or troponin.

Hereinafter, a medical instrument 800 according to an example of the present embodiment will be described using glucose as an analyte. It should be noted that for other analytes, a person skilled in the art would have been able to perform analysis on the other analytes slightly modifying the medical instrument 800 used for glucose.

FIG. 2 is a schematic diagram showing a first perspective of the medical instrument 800 according to an example of the present embodiment FIG. 3 is a schematic diagram showing a second perspective of the medical instrument 800 according to an example of the present embodiment

In some examples, the medical instrument 800 may include a sensor 820, a connection portion 840, and an attachment portion 860 (see FIG. 2 or FIG. 3). The sensor 820 may be removably mounted to the attachment portion 860. The sensor 820 may be connected to the attachment portion 860 via the connection portion 840. In other examples, the medical instrument 800 may not include the connection portion 840, in which case the sensor 820 may be directly connected to the attachment portion 860.

In some examples, the sensor 820 may be placed partially or completely subcutaneously in the host. In some examples, the sensor 820 may react with glucose in subcutaneous tissue fluid to generate host glucose information as placed subcutaneously. The attachment portion 860 may be attached to the body surface of the host. In some examples, the attachment portion 860 may also receive glucose information generated by the sensor 820. In some examples, the attachment portion 860 may also supplye the sensor 820 with the power required to acquire physiological information. Thus, continuous monitoring of the sensor 820 subcutaneously in the host can be facilitated.

In some examples, the sensor 820 may include an implant part (not shown) and a connection part (not shown). The implant part may be placed subcutaneously in the host and connected to the attachment portion 860 through the connection part. In this case, transmission of the acquired analyte information to the external device can be facilitated by placing the implant part subcutaneously in the host and connecting the implant part to the attachment portion 860 through the connection part. In some examples, the connection part may be connected to the attachment portion 860 via the connection portion 840.

In some examples, the implant part of the sensor 820 may be elongated. In some examples, the implant part may be rigid. Thus, subcutaneous placement in the host canbe facilitated In other examples, the implant part may also be flexible, in which case foreign matter sensation of the host may be reduced. In some examples, a length of the implant part can be 1 mm to 10 mm, e.g. 1 mm, 2 mm, 3 mm, 4 mm, 5 mm, 6 mm, 7 mm, 8 mm, 9 mm, or 10 mm. The implant part is implanted into the host to a depth up to the dermis layer and positioned in tissues of the skin so that the sensor 820 can collect glucose information in the interstitial fluid.

In some examples, the implant part may include a working electrode (not shown) and a counter electrode (not shown). In the present embodiment, a sensing layer comprising a glucose enzyme may be provided on the working electrode, and the sensor 820 placed subcutaneously may generate a current signal by the glucose enzyme on the working electrode performing a redox reaction with glucose in tissue fluid or blood and forming a loop with the counter electrode, and performing analysis processing on the current signal to obtain glucose concentration information In some examples, the current signal generated by the sensor 820 may be transmitted to the attachment portion 860.

In some examples, the implant portion may also include a reference electrode (not shown). In some examples, the reference electrode may form a known and fixed potential difference with tissue fluid or blood. In this case, the potential difference between the working electrode and the interstitial fluid or blood may be measured by the potential difference formed between the reference electrode and the working electrode. Thus, the voltage generated by the working electrode can be obtained more accurately. Thus, the electronic device 880 (described later) of the attachment portion 860 can automatically adjust and maintain the stabilization of the voltage at the working electrode according to a preset voltage value so that the measured current signals can more accurately reflect the glucose concentration information in the interstitial fluid or blood In some examples, the number of reference electrode(s) may be one or more, such as two.

FIG. 4 is a schematic diagram showing a connection portion 840 of the medical instrument 800 according to an example of the present embodiment. In some examples, as described above, the sensor 820 may be mounted to the attachment portion 860 via the connection portion 840. In some examples, the sensor 820 may be structurally connected to the attachment portion 860 via the connection portion 840. In some examples, the sensor 820 may also be electrically connected to the attachment portion 860 via the connection portion 840.

In some examples, the connection portion 840 may have a sensor engagement hole 842 (see FIG. 4). In some examples, when the sensor 820 is connected to the connection portion 840, an axis of the sensor 820 may pass through the sensor engagement hole 842 (see FIG. 2 or FIG. 3). In this case, the engagement of the puncture member 260 (described later) with the sensor 820 can be facilitated by the sensor engagement hole 842, thereby facilitating the placement of the sensor 820 at least partially subcutaneously in the host via the puncture member 260.

In some examples, the connection portion 840 may also have an electrical contact (or contacts) 844 (see FIG. 4). The electrical contacts) 844 can be electrically connected to the working electrode, the counter electrode, and the reference electrode of the sensor 820. Thus, it is possible to facilitate transmission of the glucose information acquired by the sensor 820 to an external device. The number of electrical contacts) 844 may be 2, 3, or 4. In the embodiment shown in FIG. 4, electrical contacts 844 may include 844a, 844b, and 844c. However, the example of the present embodiment is not limited to that, and the number of electrical contacts 844 may be the same as the number of electrodes of the sensor 820.

In some examples, the connection portion 840 may also include a structure capable of securely connecting to the attachment portion 860. In some examples, the connection portion 840 may also include a clamp portion 846 (see FIG. 4) disposed around. The clamp portion 846 of the connection portion 840 may cooperate with a clamping groove 868 (described later) of the attachment portion 860 to securely assemble the connection portion 840 to the attachment portion 860. In some examples, the clamp portion 846 may be a protrusion that protrudes outward along a periphery of the connection portion 840 (see FIG. 4). In some examples, the number of clamp portion(s) 846 may be one or more, such as one, two, three, or four. In some examples, a plurality of clamp portions 846 may be evenly distributed around the connection portion 840. In the embodiment shown in FIG. 4, the clamp portions 846 may include 846a, 846b, 846c, and 846d.

In some examples, the connection portion 840 may be substantially columnar; such as quadrangular. However; the example of the present embodiment is not limited to this, in other embodiments, the connection portion 840 may be substantially triangular hexagonal cylindrical, hemispherical, or the like. In addition, the receiving portion 866 of the attachment portion 860 may also be shaped to match the connection portion 840 to be adaptive for receiving the connection portion 840.

FIG. 5 is a schematic diagram showing an attachment portion 860 of the medical instrument 800 according to an example of the present embodiment. In some examples, the attachment portion 860 may include an attachment housing 862, a connection portion engagement hole 864, and a receiving portion 866 (see FIG. 5). A recess canbe formed in the receiving portion 866 to match the contour of the connection portion 840, and the connection portion 840 may be embedded in the receiving portion 866 (see FIG. 3).

In some examples, the attachment portion 860 may also include a hole 864 (see FIG. 5) cutting through an upper surface to a lower surface, and the axis of the sensor 820 may pass through the hole when the sensor 820 is mounted to the attachment portion 860 (see FIGS. 2 and 3). In this case, the puncture member 260 (described later) is to be punctured to the subcutaneous skin through the hole 864, thereby facilitating subcutaneous placement of the sensor 820.

In some examples, when the connection portion 840 is embedded in the receiving portion 866, the connection portion engagement hole 864 of the attachment portion 860 and the sensor engagement hole 842 of the connection portion 840 may communicate with each other. That is, when the sensor 820 is connected to the connection portion 840 and the connection portion 840 is embedded in the attachment portion 860, the central axis of the implant part of the sensor 820 may pass through the sensor engagement hole 842 and the connection portion engagement hole 864 (see FIG. 2 and FIG.3).

In some examples, the sensor 820 may be configured to be removably mounted to the attachment portion 860. In this case, the sensor 820 and the attachment portion 860 may be packaged respectively before the medical instrument 800 is received into the receiving portion 250, thereby facilitating the sensor 820 and the attachment portion 860 to be sterilized respectively in different manners.

In some examples, the attachment portion 860 may include a structure capable of securely mounting the connection portion 840. In some examples, the attachment portion 860 may also include a clamping groove 868 (see FIG. 5). A clamping groove 868 may be provided in the receiving portion 866. In some examples, the clamping groove 868 may be a recess formed on a side wall of the receiving portion 866 along a direction substantially orthogonal to the central axis of the attachment housing 862. The number of clamping grooves 868 may be the same as the number of clamp portions 846 of the connection portion 840. When the connection portion 840 is embedded in the attachment portion 860, the clamp portion 846 can be engaged with the clamping groove 868. In the embodiment shown in FIG. 5, clamping grooves 868 may include clamping groove 868a, clamping groove 868b, clamping groove 868c, and clamping groove 868d.

In some examples, electrical contacts (not shown) corresponding to the electrical contacts 844a, 844b, 844c of the connection portion 840, respectively, and communicating with the electronic device 880 (described later) are also provided on a bottom wall of the receiving portion 866. Thus, the sensor 820 is electrically connected to the electronic device 880 via the electrical contacts 844 of the connection portion 840 and the electrical contacts of the attachment portion 860.

In some examples, the attachment portion 860 is configured to adhere to the body surface of the host In some examples, the attachment portion 860 may also include an adhesive sheet 870 having adhesive property (see FIG. 2 or FIG. 3) disposed on the attachment housing 862. The attachment portion 860 may be attached and fixed to the body surface of the host by the adhesive sheet 870. In some examples, an adhesive side of the adhesive sheet 870 may be approximately equal in size to a bottom side, adjacent to the host, of the attachment housing 862. In other examples, the adhesive surface of the adhesive sheet 870 may also be slightly larger in size than the bottom surface, adjacent to the host, of the attachment housing 862.

In some examples, the attachment portion 860 may also include an electronic device 880 (described later) disposed inside the attachment housing 862. The electronic device 880 may receive glucose information generated by the sensor 820. In some examples, the electronic device 880 may further process the glucose information. In other examples, the electronic device 880 may also transmit the received glucose information to an external device, such as the reading device 900 described below. In addition, the electronic device 880 may also supply power to the sensor 820.

FIG. 6A is a schematic block diagram showing an electronic device 880 of the medical instrument 800 according to an example of the present embodiment.

In some examples, the electronic device 880 may include a power module 882, a switchmodule 884, and aprocess module 886 (see FIG. 6A). The power module 882 may supply power to the sensor 820 and/or the process module 886, the switch module 884 may control the power module 882 to power on or off the sensor 820 and/or the process module 886, and the process module 886 may process signals (e.g. glucose information) generated by the sensor 820.

In some examples, the attachment portion 860 has an initial mode configured as an open circuit and an operation mode configured as a closed circuit. In this case, the open circuit is configured when the attachment portion 860 is in the initial mode, whereby power consumption can be saved before the attachment portion 860 enters the operation state. In some examples, the switch module 884 may be in an off state when the attachment portion 860 is configured in the initial mode and may be in a closed state when the attachment portion 860 is configured in the operation mode. That is, the attachment portion 860 can be switched from the initial mode to the operation mode by the switch module 884. In this case, the switching of the attachment portion 860 from the initial mode to the operation mode is controlled by the switch module 884, whereby the power consumption before the operation can be effectively reduced

Additionally, in some examples, the electronic device 880 may also include a storage module 888 (see FIG. 6A). The storage module 888 may store signals generated by sensor 820. Additionally, in some examples, the electronic device 880 may also include a communication module 890 (see FIG. 6A). The communication module 890 may communicate with an external device (e.g., an intelligent terminal device). In some examples, the communication module 890 may be a Bluetooth module, an NFC module, a WIFI module, etc. In some examples, the intelligent terminal devices may be a smartphone, a smart watch, a tablet PC, a computer, etc.

In some examples, the electronic device 880 may transmit the glucose information generated by the sensor 820 immediately to an external device. Thus, it is possible to facilitate the processing or display of the glucose information instantly by the external device. In other examples, the glucose information generated by the sensor 820 may be received by the electronic device 880 and stored in the storage module 888 before being periodically transmitted to the external device. Thus, the power consumption due to the instant transmission can be reduced.

In some examples, the attachment portion 860 may be configured to be magnetically or optically activated from the initial mode to the operation mode. In this case, by exciting the attachment portion 860 from the initial mode to the operation mode by a non-contact action such as a magnetic action or a light action, it is able to facilitate the miniaturization of the attachment portion 860.

In some examples, the switch module 884 may be configured as an optically controlled switch For example, the switch module 884 may be in an off state in the initial mode, and when the switch module 884 is illuminated, the switch module 884 may be activated from the off state to the closed state to place the power module 882 in communication with the process module 886 to supply power to the process module 886.

In other examples, the switch module 884 may also be configured as a magnetically controlled switch For example, the switch module 884 may be in an off state in the initial mode and may be switched from the off state to the closed state when the magnetic interaction experienced by the switch module 884 changes, thereby activating the attachment portion 860 from the initial mode to the operation mode. FIG. 6B is a circuit schematic diagram showing an electronic device 880 of the medical instrument 800 according to an example of the present embodiment. Hereinafter, a switch module 884 configured to be magnetically controlled according to an example of the present embodiment will be described in detail with reference to FIG. 6B.

In some examples, the switch module 884 may be in an off state when the switch module 884 is proximate to the magnetic component and may be activated to the closed state when the switch module 884 is taken far away from the magnetic component In other examples, the switch module 884 may be in the off state when the switch module 884 is far away from the magnetic component and may be activated to the closed state when the switch module 884 is proximate to the magnetic component.

In some examples, the switch module 884 may have a Hall sensor 884a and a switch chip 884b (see FIG. 6b). In some examples, in the initial mode, the switch chip 884b may be in an off state, and when the Hall sensor 884a senses a change in magnetic action, an activation signal may be generated to activate the switch chip 884b from the off state to a closed state, thereby placing the power module 882 in communication with the process module 886. Additionally, in some examples, after the process module 886 is in communication with the power module 882, the process module 886 may also generate and send an activation signal to the switch chip 884b, thereby maintaining the switch chip 884b in a closed state.

In some examples, the applicator 1000 (described later) may include a magnet 184. Prior to the medical instrument 800 is applied to the host, the switch module 884 of the electronic device 880 may be in an off state because the electronic device 880 of the medical instrument 800 is now proximate to the magnet 184. When the medical instrument 800 is applied to the host, the switch module 884 of the electronic device 880 may be activated to the closed state because the electronic device 880 of the medical instrument 800 is now remote from the magnet 184.

Specifically, when the medical instrument 800 has not been applied to the host, the electronic device 880 may be in an initial mode at this time, i.e. the switch chip 884b of the switch module 884 may be in an off state, and the Hall sensor 884a may be proximate to a magnet 184 of the applicator 1000. When the medical instrument 800 is applied to the host, the Hall sensor 884a generates the activation signal by sensing the change of the magnetic action as a result of moving from the magnet 184 proximate to the applicator 1000 away from the magnet 184, and the switch chip 884b is activated to the closed state by the activation signal generated by the Hall sensor 884a, thereby placing the power module 882 in communication with the process module 886. Additionally, in some examples, after the power module 882 communicates with the process module 886, the process module 886 may also generate and send the activation signal to the switch chip 884b, thereby maintaining the switch chip 884b in a closed state.

Additionally, in some examples, the power module 882 of the electronic device 880 may also be configured to supply power to the sensor 820. In some examples, when the electronic device 880 is in an initial mode, the power module 882 may be disconnected from the sensor 820, and when the electronic device 880 is activated to the operation mode (i.e. the switch module 884 is in the closed state), the power module 882 may communicate with the sensor 820 to supply power to the sensor 820.

In some examples, as described above, the attachment portion 860 may be configured to communicate with the external device via wireless communication or wired communication. In this case, the analyte information acquired by the sensor 820 can be read in real time or periodically by configuring the attachment portion 860 to communicate with the external device. In some examples, the external device may be the reading device 900.

In some examples, the medical instrument 800 may be communicatively connected to the reading device 900, as described above. In some examples, the reading device 900 may be a display with communication functionality. In some examples, the communication functionality of the reading device 900 may be implemented via wireless communication or wired communication. The wireless communication means may be Bluetooth, NFC, or WIFI, etc.. The wired communication means may be USB, or optical fiber, etc..

In other examples, the reading device 900 may also be an intelligent terminal with an applicationprogiam installed to match the medical instrument 800. The intelligent terminal may be a notebook computer, tablet computer or smart phone, etc..

As described above, the medical instrument 800 according to the present embodiment can be applied to the host by the applicator 1000. Hereinafter, an applicator 1000 according to the present embodiment will be described in detail with reference to the accompanying drawings.

FIG. 7A is a schematic diagram showing an overall appearance of a first perspective of the applicator 1000 according to an example of the present embodiment FIG. 7B is a schematic diagram showing an overall appearance of a second perspective of the applicator 1000 according to an example of the present embodiment. FIG. 7C is an exploded schematic diagram showing the applicator 1000 according to an example of the present embodiment. In FIGS. 7A, 7B, and 7C, the line CA schematically represents the central axis of the applicator 1000.

In the present embodiment, the applicator 1000 may include a housing 10 that can provide a moving space, and an auxiliary mechanism 20 (see FIGS. 7A, 7B, and 7C).configured to be movable within the moving space of the housing 10 The housing 10 may have a proximal end that is proximal to the host and a distal end that is distal to the host during operation. The auxiliary mechanism 20 may be releasably held by the housing 10 and may accommodate a medical instrument 800. The auxiliary mechanism 20 may be configured to move relatively to the housing 10 when released and driven toward the host to apply the medical instrument 800 to the host. In this disclosure, "proximal end" can be understood as the end that is proximal to the host in operation and "distal end" can be understood as the end that is distal to the host in operation.

In some examples, the housing 10 may have a moving space and the auxiliary mechanism 20 may be releasably held by the housing 10. In some examples, the auxiliary mechanism 20 may move within the moving space of the housing 10 when released Additionally, in some examples, the housing 10 may also define an attachment position for the medical instrument 800 on the body surface of the host.

In some examples, the housing 10 may comprise a first housing 100 and a second housing 140. The first housing 100 and the second housing 140 may be assembled together to form a moving space. The first housing 100 may releasably hold the auxiliary mechanism 20. The second housing 140 may define an attachment position of the medical instrument 800 on the body surface of the host and may define a movement path of the auxiliary mechanism 20. The auxiliary mechanism 20, when released by the first housing 100, may move relatively to the second housing 140 following the movement path defined by the second housing 140 and apply the medical instrument 800 to the attachment position defined by the second housing 140. In the present embodiment, the axis of the first housing 100 and the axis of the second housing 140 may be substantially parallel to the central axis CA of the applicator 1000.

In some examples, the auxiliary mechanism 20 may include a moving body 200, a receiving portion 250, and a puncture member 260 (see FIG. 7C). The moving body 200 may be releasably held by the first housing 100, and the moving body 200 may be configured to move following the movement path defined by the second housing 140 when released The receiving portion 250 may be provided on the moving body 200 and may be configured to receive and accomodate the medical instrument 800, and the receiving portion 250 may be driven toward the attachment position defined by the second housing 140. The puncture member 260 may be provided on the moving body 200 passing through the receiving portion 250. The moving body 200 can move toward the proximal end of the housing 10 once released, and the receiving portion 250 and the puncture member 260 also move toward the proximal end of the housing 10 so as to apply the medical instrument 800 received in the receiving portion 250 to the host. In some examples, the puncture member 260 may be punctured subcutaneously in the host, thereby placing the medical instrument 800 at least partially subcutaneously in the host

Additionally, in some examples, the applicator 1000 may also include a first drive mechanism 30 (see FIG. 7C). The first drive mechanism 30 may be configured to act on the moving body 200 toward the proximal end When released, the moving body 200 may be driven toward the proximal end by the first drive mechanism 30 to push the medical instrument 800 received in the receiving portion 250 toward the host, for example, to the attachment position defined by the second housing 140. In addition, the medical instrument 800 may be placed at least partially subcutaneously in the host by the puncture member 260.

In other examples, the applicator 1000 may not include the first drive mechanism 30. In this case, when released, the moving body 200 may also be manually driven to move toward the proximal end.

Additionally, in some examples, the puncture member 260 may be releasably disposed on the moving body 200, and the puncture member 260 may be configured to move relatively to the receiving portion 250 when released. For example, the puncture member 260 may move in a direction away from the host relatively to the receiving portion 250 when released.

Additionally, in some examples, the applicator 1000 may also comprise a second drive mechanism 40 (see FIG. 7C). The second drive mechanism 40 may be configured to act on the puncture member 260 toward the distal end When released, the puncture member 260 may be driven toward the distal end by the second drive mechanism 40 to enable the puncture member 260 to move away from the host.

In other examples, the applicator 1000 may not include the second drive mechanism 40. In this case, the puncture member 260 may be manually driven away from the host. For example, in some instances, the puncture member 260 may also be integrally connected with the moving body 200, may be moved away from the host by manually movement of the moving body 200 away from the host.

In some examples, as described above, the housing 10 may include a first housing 100 (see FIGS. 7A, 7B, or 7C). In some examples, the first housing 100 may include a peripheral portion 110 and an end portion 120 (see FIG. 7C).

FIG. 8A is a schematic perspective diagram showing a peripheral portion 110 of the first housing 100 according to an example of the present embodiment. FIG. 8B is a schematic perspective diagram showing a first perspective of the end portion 120 of the first housing 100 according to an example of the present embodiment. In some examples, the peripheral portion 110 may be formed as a cylindrical shell that is hollow and a topto-bottom through-hole along the central axis CA of the applicator 1000 (see FIG. 8A), and the end portion 120 may be provided on the peripheral portion 110 near the distal end of the housing 10. In some examples, the central axis CA may pass through the geometric center of end portion 120.

In some examples, one or more rib shape protrusions may be provided on an inner wall of the first housing 100, and one or more rib shape grooves may be provided on an outer wall of the second housing 140. In some examples, when the second housing 140 is mounted to the first housing 100, one or more rib shape protrusions of the first housing 100 may be respectively embeded into one or more rib shape grooves of the second housing 140. In this case, the assembly of the first housing 100 and the second housing 140 can be facilitated by the matching of the rib shape protrusions and the rib shape grooves.

In some examples, a peripheral rib 112 (see FIG. 8A) extending substantially in the direction of the central axis CA may be provided on an inner wall of the peripheral portion 110. In some examples, the number of peripheral ribs 112 may be multiple, for example, 2, 3, or 4. In the embodiment shown in FIG. 8A, the number of the peripheral ribs 112 is four, and the peripheral ribs 112 may include a peripheral rib 112a, a peripheral rib 112b, a peripheral rib 112c, and a peripheral rib 112d In some examples, peripheral rib 112 may act as a stiffening rib to effectively increase the deformation resistance of peripheral portion 110. In some examples, the peripheral rib 112 may also act as a guide rib to facilitate assembly of the first housing 100 with the second housing 140 (described later).

In some examples, connection columns 114 may also be provided on the inner wall of the peripheral portion 110 substantially parallel to the central axis CA (see FIG. 8A). In some examples, the number of connection columns 114 may be multiple, such as 2, 3, or 4. In the embodiment shown in FIG. 8A, the number of the connection columns 114 is three, and the connection columns 114 may include a connection column 114a, a connection column 114b, and a connection column 114c. In some examples, the end of the connection column 114 near the proximal end of the applicator 1000 can be a hot type column end, i.e. it can be melted as heated up and then can solidify as cooled down. In this case, the connection column 114 may be assembled with the second housing 140 in cooperation with a hole provided on the second housing 140 (to be described later).

In some examples, the end portion 120 may include a substantially cylindrical end body 121 (see FIG. 8B).

In some examples, the first housing 100 may include a first holding portion 130. In some examples, the first holding portion 130 may be configured to releasably hold the auxiliary mechanism 20. In some examples, the first holding portion 130 may be disposed at the end portion 120 (see FIG. 8B). In some examples, the first holding portion 130 may extend from the end body 121 along the central axis CAin a manner toward the proximal end of the applicator 1000. In this case, when the auxiliary mechanism 20 is held by the end portion 120, the auxiliary mechanism 20 may be located in the hollow portion of the peripheral portion 110, and when released, the auxiliary mechanism 20 may move substantially in the axial direction of the peripheral portion 110. That is, when released, the auxiliary mechanism 20 may move substantially along the central axis CA.

In some examples, the first holding portion 130 may be releasably interlocked with a held part (eg. the first locking portion 236 of the moving body 200, which will be described later) by at least one structure of a buckle, a hook, a latch, or a pin. In this case, the first holding portion 130 and the first locking portion 236 are releasably interlocked by at least one structure of a buckle, a hook, a latch, or a pin, thereby providing a easily releasable interlock mean

In some examples, the number of first holding portions 130 may be one or more, such as 1, 2, 3, or 4. In the embodiment shown in FIG. 8B, the number of the first holding portions 130 may be two, and the first holding portions 130 may include a first holding portion 130a and a first holding portion 130b. The first holding portion 130a and the first holding portion 130b may be disposed equidistantly on opposite sides of the central axis CA (see FIG. 8B).

In some examples, the first holding portion 130 may have a surface facing the distal end, and the first locking portion 236 may have a surface facing the proximal end, and the surface, facing the distal end, of the first holding portion 130 and the surface, facing the proximal end, of the first locking portion 236 may engage each other when the moving body 200 is held In some examples, the surface, facing the distal end, of the first holding portion 130 and the surface, facing the proximal end, of the first locking portion 236 may be split from each other when the moving body 200 is released.

In some examples, at least a portion of the first holding portion 130 may be "L"-shaped or hook-shaped. Thus, it is possible to easily form a buckle or the like to interlock with the first locking portion 236.

In some examples, the first holding portion 130 may include an arm 132 extending substantially in the direction of the central axis CA, and a protrusion 134 (see FIG. 8B) linked with the arm 132 and projecting toward the central axis CA in a direction substantially orthogonal to the central axis CA. In some examples, the arms 132 of the first holding portion 130 may extend outwardly from the end body 121. In this case, when the protrusion 134 of the first holding portion 130 is overlapped with the first locking portion 236, the protrusion 134 is actuated by actuating the arm 132, whereby the first holding portion 130 can easily release the first locking portion 236.

Additionally, in some examples, a groove (not shown) may be provided on the arm 132 of the first holding portion 130 substantially along the central axis CA. In some examples, the groove may face the central axis CA. In this case, an actuation portion 504 of the first trigger mechanism 50 (to be described later) can move along the groove and actuate the arm 132 of the first holding portion 130.

In some examples, the arm 132 of the first holding portion 130 may be resilient. In some examples, the arm 132 of the first holding portion 130 may be resilient in a direction substantially orthogonal to the central axis CA. In some examples, the arm 132 of the first holding portion 130 may tend to contract toward the central axis CA in a direction from the distal end to the proximal end That is, in a natural state (i.e. a state where no external force is applied), the end, near the proximal end, of the arm 132 is closer to the central axis CA than the end, near the distal end, of the arm 132. In other examples, the arm 132 may also be substantially parallel to the central axis CA. In some examples, the arm 132 may pivot when acted upon in a direction substantially orthogonal to central axis CA. For example, when the arm 132 is acted upon in a direction substantially orthogonal to the central axis CA in a manner away from the central axis CA, the arm 132 may pivot such that the end, near the proximal end, of the arm 132 moves in a direction away from the central axis CA. Thus, the held component can be easily released by operation.

In some examples, the arm 132 of the first holding portion 130 may converge toward the central axis CA in a directionfromthe distal end to the proximal end, and the protrusion 134 of the first holding portion 130 may protrude toward the central axis CA. In this case, the first locking portion 236 can be held on the inner side (close to the central axis CA) by the first holding portion 130 through entirely inwardly folding, and the first locking portion 236 can be released by actuating the first holding portion 130 toward the outer side (away from the central axis CA), whereby the holding and releasing of the first locking portion 236 can be facilitated.

In other examples, the arm 132 of the first holding portion 130 may back-face away from the central axis CA in a direction from the distal end to the proximal end, and the protrusion 134 of the first holding portion 130 may protrude with backing-face away from the central axis CA. In this case, the first locking portion 236 can be held at the outside (away from the central axis CA) by dispersing the first holding portion 130 as a whole outwardly, and the first locking portion 236 can be released by actuating the first holding portion 130 inwardly (close to the central axis CA), whereby the holding and releasing of the first locking portion 236 can be facilitated.

However, the examples of the present embodiment are not limited to this, and the conception of the present invention is that the first holding portion 130 forms a structure such as a buckle, a hook, a latch, or a pin to hold the held component, and releases the held component by moving the buckle, the hook, the latch, or the pin away from the original position, and based on this conception, the arm 132 of the first holding portion 130 and the protrusion 134 of the first holding portion 130 may converge or protrude in other directions in case that the buckle can be formed.

In some examples, the protrusion 134 of the first holding portion 130 may have a surface facing the distal end In some examples, the surface, facing the distal end, of the protrusion 134 may be substantially orthogonal to the central axis CA. The held component(e.g. the first locking portion 236 of the moving body 200 to be described later), may be held and not moved toward the proximal end by overlapping/abutting the surface, facing the distal end, of the protrusion 134. The held component will be released when the arm 132 of the first holding portion 130 is actuated so that the surface, facing the distal end, of the protrusion 134 leaves the original position, i.e. no longer abuts against the held component

That is, the first holding portion 130 may be a buckle, a hook, a latch, a pin, or other structures formed on the first housing 100 (e.g. the end portion 120 of the first housing 100). For example, the first holding portion 130 may be formed in a finger shape, a straight line, an "L" shape, a "J" shape, a "Z" shape, and other structures which can be divided into at least two parts, at least one part of which (e.g. an arm attached to the first housing 100) may pivot, and another part of which (e.g. a protrusion protruding outwardly from the arm and abutting the held component) may leave the original position as it pivots, thereby the hooking, hanging, latching, pushing, supporting of the held component can be released

FIG. 9A is an exploded schematic diagram showing the first holding portion 130, the moving body 200, and the first drive mechanism 30 according to an example of the present embodiment FIG. 9B is a schematic cross-sectional diagram showing a state as the moving body 200 is held according to an example of the present embodiment FIG. 9C is a schematic cross-sectional diagram showing a state ase the moving body 200 is released according to an example of the present embodiment. It should be noted that FIG. 9B and FIG. 9C mainly illustrate the holding and releasing of the moving body 200 by the first holding portion 130 without illustrating the first drive mechanism 30 and other components.

In some examples, as described above, the auxiliary mechanism 20 may comprise the moving body 200, and the receiving portion 250 provided on the moving body 200. The moving body 200 may be releasably held on the first housing 100. In some examples, the moving body 200 may be releasably held by the first holding portion 130. The moving body 200 can be driven toward the proximal end after being released to push the medical instrument 800 accommodated in the receiving portion 250 toward the host.

In some examples, a first driving portion 30 may be provided between the moving body 200 and the first housing 100 (see FIG. 9A). The first driving portion 30 may act on the moving body 200 toward the proximal end. After being released, the moving body 200 can be driven toward the proximal end by the first driving portion 30 to push the medical instrument 800 accommodated in the receiving portion 250 toward the host.

In other examples, as described above, the applicator 1000 may not include the first driving portion 30. In this case, when released, the moving body 200 can also be pushed toward the host by human power thereby pushing the medical instrument 800 accommodated in the receiving portion 250 toward the host.

In some examples, the moving body 200 may include a first bottom portion 210, a second bottom portion 220, and a side wall 230 connecting the first bottom portion 210 and the second bottom portion 220 (see FIG. 9A). The first bottom portion 210 may be near the distal end, the second bottom portion 220 may be near the proximal end, and the receiving portion 250 may be disposed at the second bottom portion 220.

In some examples, a hollow portion may be formed between the first bottom portion 210, the second bottom portion 220, and the side wall 230. In some examples, the puncture member 260 may be releasably disposed within the hollow portion of the moving body 200. In some examples, the puncture member 260 may move along the hollow portion of the moving body 200 when released. In this case, the hollow portion is formed by the first bottom portion 210, the second bottom portion 220, and the side wall 230, and the receiving portion 250 is provided to the second bottom portion 220, and the puncture member 260 is provided to the hollow portion, whereby it is possible to facilitate the movement of the puncture member 260 relative to the receiving portion 250 when the puncture member 260 is released. Thus, when the medical instrument 800 is applied to the host, the puncture member 260 is released and moved relatively to the receiving portion 250, thereby enabling the puncture member 260 to exit the host.

In other examples, the puncture member 260 may also be fixedly disposed on the moving body 200. In this case, when the medical instrument 800 is applied to the host, the moving body 200 can integrally leave the host by means of human power, whereby the puncture member 260 can also leave the host.

In some examples, the first drive mechanism 30 may be disposed between the first bottom portion 210 and the first housing 100. In some examples, a positioning portion 212 for positioning the first drive mechanism 30 may be provided on the first bottom portion 210 (see FIG. 9A). In this case, the position where the first drive mechanism 30 acts on the moving body 200 can be more stably limited by the positioning portion 212.

In some examples, the positioning portion 212 of the first bottom portion 210 may be a columnar protrusion projecting toward the distal end from the first bottom portion 210 along the central axis CA (see FIG. 9A). In some examples, the positioning portion 212 may be cylindrical. In some examples, the positioning portion 212 may be solid cylindrical, and the first drive mechanism 30 may be sheathed around an outer periphery of the positioning portion 212. In other examples, the positioning portion 212 may be hollow cylindrical, and the first drive mechanism 30 may be sheathed around the outer periphery of the positioning portion 212 or may be embedded within an inner periphery of the positioning portion 212.

In some examples, the first drive mechanism 30 may have an energy storage state and an energy release state, and as the first drive mechanism 30 is switched from the energy storage state to the energy release state, the first drive mechanism 30 may release energy and act on the moving body 200 in the manner toward the proximal end When the moving body 200 is held, the first drive mechanism 30 may be in the energy storage state and located between the moving body 200 and the first housing 100, and when the moving body 200 is released, the first drive mechanism 30 may switch from the energy storage state to the energy release state and act on the moving body 200 in the manner toward the proximal end

In some examples, the first drive mechanism 30 may have a compressed state and an expanded state, and the first drive mechanism 30 may release energy when the first drive mechanism 30 is switched from the compressed state to the expanded state. In some examples, the first drive mechanism 30 may be a compressible, resilient member. In some examples, the first drive mechanism 30 may be a spring. In some examples, one end of the first drive mechanism 30 may be disposed enclosing the positioning portion 212 of the first bottom portion 210.

In some examples, the end portion 120 may have a positioning portion 122 (see FIG. 8B). In some examples, the positioning portion 122 may be of cylindrical shape. In this case, the other end of the first drive mechanism 30 may be disposed enclosing the positioning portion 122. In some examples, the positioning portion 122 may be of hollow cylindrical shape. In this case, the other end of the first drive mechanism 30 may be disposed enclosing the positioning portion 122 or embedded in the positioning portion 122. In some examples, the positioning portion 122 may be comprised of at least two segments of arcuate post For example, in the embodiment shown in FIG. 8B, the positioning portion 122 may include arcuate post 122a and arcuate post 122b. In this case, the other end of the first drive mechanism 30 may be embedded between the arcuate post 122a and the arcuate post 122b.

In addition, the applicator 1000 of the present embodiment is not restricted thereto. In some examples, the applicator 1000 may also not include the first drive mechanism 30. In this case, when the moving body 200 is released, the moving body 200 can also be moved toward the proximal end by manually applying an action to the moving body 200.

In some examples, the moving body 200 may include a first locking portion 236 configured to releasably interlock with the first holding portion 130. Thus, the moving body 200 canbe releasably held by the first holding portion 130 through the cooperation of the first locking portion 236 and the first holding portion 130.

In some examples, the first locking portion 236 may be provided on a side wall 230 of the moving body 200. That is, the first locking portion 236 may be disposed on the side wall 230 of the moving body 200 (see FIG. 9A). The first locking portion 236 may cooperate with the first holding portion 130 to hold the moving body 200 to the first housing 100.

In some examples, the number of first locking portion(s) 236 may be one or more, such as 1, 2, 3, or 4. In some examples, the number of first locking portions 236 may be the same as the number of first holding portions 130. In the embodiment shown in FIG. 9A, the number of the first locking portions 236 is two, and the first locking portions 236 may include a first locking portion 236a and a first locking portion 236b.

In some examples, the first locking portion 236 may be formed as a clamp structure. Specifically, the first locking portion 236 may have a surface facing the proximal end, the first holding portion 130 may hold the moving body 200 by clamping, abutting or engaging with the surface, and the first holding portion 130 may release the moving body 200 by leaving the surface. That is, the first holding portion 130 and the first locking portion 236 may be formed in an interlocking structure, a projection of the first holding portion 130 and the first locking portion 236 in a direction along the central axis CA has an overlapping portion, the first holding portion 130 holds the first locking portion 236 through the overlapping portion, and the first holding portion 130 and/or the first locking portion 236 may enable the first holding portion 130 to release the first locking portion 236 by moving away from each other to cancel the overlapping portion In some examples, the first holding portion 130 and/or the first locking portion 236 may be actuated in a manner facing away from each other such that the first locking portion 236 is released by the first holding portion 130.

In some examples, the first locking portion 236 may be substantially parallel to the central axis CA or facing away from the central axis CAin a direction from the proximal end to the distal end. In this case, the first locking portion 236 may be closer to the central axis CA than the first holding portion 130, thereby facilitating cooperation with the inwardly folded first holding portion 130 for interlocking.

In other examples, the first locking portion 236 may be substantially parallel to the central axis CA or close toward the central axis CA in a direction from the proximal end to the distal end In this case, the first locking portion 236 may be farther away from the central axis CAthan the first holding portion 130, thereby being able to facilitate cooperation with the outwardly dispersed first holding portion 130 for interlocking.

In some examples, the first locking portion 236 may be formed in such a manner as to be recessed from an outer surface of the side wall 230 toward the central axis CA. In some examples, the first locking portion 236 may be formed as a hollow structure, such as a through hole extending through the side wall 230 in a direction substantially orthogonal to the central axis CA. In some examples, the first locking portion 236 may also be formed in such a manner that a peripheral edge of the first bottom portion 210 protrudes from the side wall 230 in a direction orthogonal to the central axis CA, in this case the first bottom portion 210 and the side wall 230 may be fixedly connected to each other. In other examples, the first locking portion 236 may also be of an inverted hook or "L" shape.

The example of the present embodiment is not restricted thereto, and as described above, the idea of the present invention is to form the first holding portion 130 and the first locking portion 236 into a buckle, a hook, a latch, a pin or other structures for interlocking, in which case the first locking portion 236 may have a surface facing the proximal end of the applicator 1000, and to hold the first locking portion 236 by the first holding portion 130 through engaging the surface, facing the proximal end, of the first locking portion 236 and the surface, facing the distal end, of the first holding portion 130 with each other. The first holding portion 130 releases the first locking portion 236 by separating the surface, facing the proximal end, of the first locking portion 236 from the surface, facing the distal end, of the first holding portion 130.

With reference to FIG. 9B and FIG. 9C, in the embodiment shown in FIG. 9B and FIG. 9C, the first holding portion 130 may be formed in "L" shape, and the first locking portion 236 may be formed in a hollow structure provided on the side wall 230. When the moving body 200 is held, that is, as shown in FIG. 9B, the protrusion 134 of the first holding portion 130 is located in the hollow structure of the first locking portion 236, thereby supporting the moving body 200 to hold the moving body 200. When the moving body 200 is released, i.e. as shown in FIG. 9C, the arm 132 of the first holding portion 130 pivots in a direction away from the central axis CA and moves the protrusion 134 away from the central axis CA, the protrusion 134 moves away from the hollow structure of the first locking portion 236, so that the moving body 200 is released and moves toward the proximal end under actuation of the first drive mechanism 30.

In addition, with reference to FIG. 9B and FIG. 9C, in the embodiment shown in FIG. 9B and FIG. 9C, the arm 132 of the first holding portion 130 may converge toward the central axis CA in a direction from the distal end to the proximal end In this case, by actuating the arm 132 of the first holding portion 130 in the direction of the central axis CA, the arm 132 is pivoted and the protrusion 134 is moved away from the central axis CA, and the protrusion 134 is moved away from the hollow structure of the first locking portion 236, so that the moving body 200 is released.

Additionally, in some examples, the applicator 1000 may further include a first trigger mechanism 50 (see FIG. 7C) configured to separate the first holding portion 130 from the first locking portion 236 to release the moving body 200. Thereby, the moving body 200 can be easily released by the first trigger mechanism 50.

In some examples, the applicator 1000 may also include the first trigger mechanism 50 (see FIG. 7C). The first trigger mechanism 50 is configured to trigger the first holding portion 130 so that the moving body 200 is released by the first holding portion 130. In some examples, the first trigger mechanism 50 may be configured to actuate the first holding portion 130 and/or the first locking portion 236 away from each other to release the moving body 200. For example, the first trigger mechanism 50 may be configured to actuate the first holding portion 130 to move away from the central axis CAto release the holding of the first locking portion 236 so that the movingbody 200 is released. That is, the first trigger mechanism 50 may be configured to actuate the first holding portion 130 and/or the first locking portion 236 to move away from each other such that the first locking portion 236 is released.

FIG. 10A is an exploded schematic diagram showing the end portion 120 of the first housing 100 and the first trigger mechanism 50 according to an example of the present embodiment; FIG. 10B is a perspective diagram showing a second perspective of the end portion 120 of the first housing 100 according to an example of the present embodiment; FIG. 10C is a schematic bottom diagram showing the end portion 120 of the first housing 100 according to an example of the present embodiment; FIG. 10D is a schematic top diagram showing the end portion 120 of the first housing 100 according to an example of the present embodiment; FIG. 10E is a schematic perspective diagram showing the first trigger mechanism 50 according to an example of the present embodiment; FIG. 10F is a schematic bottom diagram showing the first trigger mechanism 50 according to an example of the present embodiment; FIG. 10G is a schematic cross-sectional diagram showing the first trigger mechanism 50 according to an example of the present embodiment before being mounted to the end portion 120; FIG. 10H is a schematic cross-sectional diagram showing the first trigger mechanism 50 according to an example of the present embodiment after being mounted to the end portion 120.

In some examples, the first trigger mechanism 50 may comprise a pressing portion 502, an actuation portion 504, and a mounting portion 506 (see FIG. 10A). The first trigger mechanism 50 may be mounted to the end portion 120 via the mounting portion 506. By acting on the pressing portion 502, the actuation portion 504 can actuate the first holding portion 130, so that the first holding portion 130 releases the holding of the moving body 200 so that the moving body 200 is released. In some examples, the pressing portion 502 may be substantially pie-shaped.

In some examples, the actuation portion 504 of the first trigger mechanism 50 may extend substantially in the direction of the central axis CA. In some examples, the first trigger mechanism 50 is configured to be movable in the direction of the central axis CA. In this case, by driving the actuation portion 504 of the first trigger mechanism 50 along the central axis CA of the applicator 1000, the first holding portion 130 can be actuated to be separated from the first locking portion 236 with simple operation.

In some examples, the actuation portion 504 of the first trigger portion 50 may at least partially coincide with the first holding portion 130 (e.g. at least partially coincide with the arm 132 of the first holding portion 130) as projected in the direction along the central axis CA, and the first holding portion 130 may be actuated away from the first locking portion 236 as the first trigger portion 50 moves in the direction of the central axis CA. Thus, the first locking portion 236 can be released from the first holding portion 130 by a simple operation.

In other examples, the actuation portion 504 of the first trigger portion 50 may at least partially coincide with the first locking portion 236 as projected in the direction along the central axis CA, and the first locking portion 236 may be actuated away from the first holding portion 130 as the first trigger portion 50 moves in the direction of the central axis CA. Thus, the first locking portion 236 can be easily released by the operation.

In some examples, the number of mounting portion(s) 506 may be one or more, such as 1, 2, 3, or 4. In the embodiment shown in FIG. 10E, the mounting portions 506 may be mounting portion 506a and mounting portion 506b. In some examples, the plurality of mounting portions 506 may be symmetrically distributed about a periphery of the central axis CA. In the embodiment shown in FIG. 10E, the mounting portion 506a and mounting portion 506b are symmetrically distributed on both sides of the central axis CA. In some examples, the mounting portions 506 may be formed at a periphery of the pressing portion 502.

In some examples, the mounting portion 506 may extend from the pressing portion 502 toward the proximal end substantially along the central axis CA. In some examples, the mounting portion 506 may have an arm 512 substantially along the direction of the central axis CA, and a protrusion 514 protruding from the arm 512 toward the central axis CA along the direction substantially orthogonal to the central axis CA (see FIG. 10E). In some examples, the arm 512 of the mounting portion 506 may extend toward the proximal end from the pressing portion 502 substantially along the central axis CA.

In some examples, the arm 512 of the mounting portion 506 may be resilient. In some examples, the arm 512 of the mounting portion 506 may tend to contract toward the central axis CA in the direction from the distal end to the proximal end That is, in a natural state (i.e. a state where no external force is applied), the end, near the proximal end, of the arm 512 is closer to the central axis CA than the end, near the distal end, of the arm 512. In other examples, the arm 512 may also be substantially parallel to the central axis CA. In some examples, arm 512 may pivot when acted upon in the direction substantially orthogonal to central axis CA. For example, when the arm 512 is acted upon toward the central axis CAin the direction substantially orthogonal to the central axis CA, the arm 512 may pivot such that the proximal end of the arm 512 moves away from the central axis CA.

In some examples, the protrusion 514 of the mounting portion 506 may have a surface facing the distal end. In some examples, the surface facing the distal end, of the protrusion 514 may be substantially orthogonal to the central axis CA. Components cooperating with the mounting portion 506 (such as an offset portion 124 and an engagement portion 126 of the end portion 120 to be described later) may be fitted into engagement with the mounting portion 506 by clamping/abutting the surface facing the distal end of the protrusion 514.

That is, the mounting portion 506 may be a clamp or a latch structure formed on the first trigger mechanism 50. For example, the mounting portion 506 may be formed as shape of finger straight line, "L" , "J", "Z", and other structures which can be divided in to at least two parts, wherein at least one part (e.g. the arm 512 connected to the pressing portion 502) may pivot, and the other part (e.g. the protrusion 514 protruding outward from the arm 512 and abutting against the fitting engagement member) may fittingly engage with, , e.g. be hooked, hung, latched, top, supported, etc. by the fitting engagement member (e.g. the offset portion 124 and the engagement portion 126 of the end portion 120 to be described later) as it pivots.

In some examples, the end portion 120 may comprise the offset portion 124 and the engagement portion 126 (see FIG. 10B and FIG. 10C). In some examples, there may be a gap between the offset portion 124 and the engagement portion 126. In this case, the mounting portion 506 may enter the fitting position via the gap between the offset portion 124 and the engagement portion 126. In some examples, the number of offset portion(s) 124 and engagement portion(s) 126 of the end portion 120 may be multiple, such as 1, 2, 3, or 4. In some examples, the number of offset portions 124 of the end portion 120, as well as the number of the engagement portions 126, may be the same as the number of mounting portions 506. In the embodiment shown in FIG. 10B and FIG.10C, the offset portions 124 may be offset portions 124a and 124b, and engagement portions 126 may be 126a and 126b.

In some examples, the distance between the offset portion 124 and the central axis CA is greater than the distance between the engagement portion 126 and the central axis CA In some examples, the offset portion 124 may gradually approach the central axis CA in the direction along the central axis CA toward the proximal end In some examples, an end near the proximal end of the offset portion 124 may meet or slightly coincide with the engagement portion 126, projected in the direction along the central axis CA.

Now with reference to FIG. 10G and FIG. 10H, the fitting engagement of the mounting portion 506 with the offset portion 124 and the engagement portion 126 is further described. Prior to the fitting engagement, The distance between the projection of the mounting portion 506a and the projection of the mounting portion 506b may be slightly smaller than or substantially equal to the distance between the engagement portion 126a and the engagement portion 126b (see FIG. 10G) in the direction substantially orthogonal to the central axis CA. After fitting engagement, i.e. being moved along the central axis CA toward the proximal end, the mounting portion 506 may pass through the gap between the offset portion 124 and the engagement portion 126, and the arm 512 of the mounting portion 506 is pressed by the offset portion 124 toward the central axis CA such that the distance between the protrusion of the mounting portion 506a and the protrusion of the mounting portion 506b gradually decreases and is less than the distance between the engagement portion 126a and the engagement portion 126b. In this case, the surface facing the distal end of the protrusion 514 of the mounting portion 506 may engage the engagement portion 126 under the pressing action of the offset portion 124, thereby interlocking the mounting portion 506 with the engagement portion 126 to fitly engage the first trigger mechanism 50 with the end portion 120 (see FIG. 10H).

That is, the first trigger mechanism 50 and the end portion 120 may be fitted and engaged by a clamp, a latch, or the like.

Additionally, in some examples, the first trigger mechanism 50 may also have a rib groove 507. The end portion 120 may have an end rib 128. In some examples, a depth of the rib groove 507 may be approximately the same as a thickness of the end rib 128, and a width of the rib groove 507 may be approximately the same as a width of the end rib 128. In this case, a guiding effect on the fitting process can be provided by the cooperation of the rib groove 507 with the end rib 128. Additionally, undesired rotation of the first trigger mechanism 50 mounted on the end portion 120 is also effectively suppressed by the engagement of the rib groove 507 with the end rib 128.

In some examples, the rib groove 507 and the end rib 128 may extend substantially in the direction of the central axis CA. In some examples, the number of rib groove(s) 507 may be one or more, such as 1, 2, 3, or 4. For example, in the embodiment shown in FIG. 10E and FIG. 10F, the rib grooves 507 may be rib groove 507a, rib groove 507b, rib groove 507c, and rib groove 507d. Accordingly, the number of end rib(s) 128 may be one or more, and may be the same as the number of rib grooves 507. In the embodiment shown in FIG. 10B, the end ribs 128 may be end rib 128a, end rib 128b, end rib 128c, and end nbs128d.

FIG. 10I is a schematic cross-sectional diagram showing the first holding portion 130 according to an example of the present embodiment before being activated by the first activation mechanism 50; FIG. 10J is a schematic cross-sectional diagram showing the first holding portion 130 according to an example of the present embodiment after being triggered by the first trigger mechanism 50. It should be noted that FIG. 10I and FIG. 10J mainly illustrate the triggering of the first holding portion 130 by the first trigger mechanism 50.

In some examples, as described above, the actuation portion 504 of the first trigger mechanism 50 may actuate the first holding portion 130 so that the first holding portion 130 releases the holding of the moving body 200, thereby allowing the moving body 200 to be released.

In some examples, the actuation portion 504 may be formed as a columnar structure extending from the pressing portion 502 substantially along the central axis CAtoward the proximal end (see FIG. 10E). In some examples, the number of actuation portion(s) 504 may be one or more, such as 1, 2, 3, or 4. In some examples, the number of the actuation portions 504 may be the same as the number of the first holding portions 130. In the embodiment shown in FIG. 10E, the actuation portions 504 may include an actuation portion 504a and an actuation portion 504b.

In some examples, a through structure corresponding to the first holding portion 130 is formed on the end portion 120 in the direction substantially along the central axis CA (see FIG. 10B, FIG. 10C, or FIG. 10D). In some examples, a projection plane of the through structure may cover a projection plane of the actuation portion 504 as projected in the direction along the central axis CA.

Hereinafter, the activation of the first holding portion 130 by the actuation portion 504 will be described in detail with reference to FIG. 10I and FIG. 10J. As described above, the first holding portion 130 gradually converges toward the central axis CA in a direction toward the proximal end along the central axis CA. The actuation portion 504 is configured to actuate the first holding portion 130 such that one end near the proximal end of the first holding portion 130 moves away from the central axis CA, thereby releasing the holding of the moving body 200. In some examples, the distance between the actuation portion 504a and the actuation portion 504b may be slightly greater than the distance between the first holding portion 130a and the first holding portion 130b in the direction substantially orthogonal to the central axis CA.

Before the first holding portion 130 is actuated by the actuation portion 504, one ends, near the proximal end of the first holding portion 130a and the first holding portion 130b converge to interlock with the first locking portion 236 to hold the moving body 200 (see FIG. 10I). When the first holding portion 130 is actuated by the actuation portion 504 (e.g. by pressing the pressing portion 502 in the direction toward the proximal end), the first holding portion 130a and the first holding portion 130b gradually splay outwardly (i.e. away from the central axis CA) under the actuation of the actuation portion 504, so that the protrusion 134 of the first holding portion 130 moves away from the first locking portion 236, thereby releasing the interlock between the first holding portion 130 and the first locking portion 236, so that the moving body 200 is released (see FIG. 10J).

FIG. 11A is a schematic perspective diagram showing a second embodiment of the end portion 120 according to an example of the present embodiment, FIG. 11B is a schematic top diagram showing the second embodiment of the end portion 120 according to an example of the present embodiment, FIG. 11C is a schematic bottom diagram showing a second embodiment of the first trigger mechanism 50 according to an example of the present embodiment; FIG. 11D is a schematic bottom diagram showing a third embodiment of the first trigger mechanism 50 according to an example of the present embodiment.

In some examples, the end body 121 may be a solid structure. That is, the end body 121 may not have a through hole. In some examples, the end portion 120 may have a positioning portion 122 extending from the end body 121 toward the proximal end (see FIG. 8B). In this case, one end of the first drive mechanism 30 may be disposed enclosing the positioning portion 212 of the moving body 200, and the other end of the first drive mechanism 30 may be disposed enclosing the positioning portion 122 of the end portion 120. In this manner the first drive mechanism 30 is disposed between the moving body 200 and the end portion 120.

In some examples, the first drive mechanism 30 may be a spring. Thus, it is able to provide an actuation mechanism for the moving body 200 through a simplified structural design.

In other examples, the end body 121 may also be a through structure (see FIGS. 11A and 11B). That is, the end portion 120 may have a through hole 129. In some examples, an aperture of the through hole 129 may be approximately a diameter of the first drive mechanism 30. In this case, one end of the first drive mechanism 30 may abut against the moving body 200, and the other end of the first drive mechanism 30 may abut against the first trigger mechanism 50. In some examples, the first trigger mechanism 50 may have a positioning portion 508 (see FIG. 10C), one end of the first drive mechanism 30 may be disposed enclosing the positioning portion 212 of the moving body 200, and the other end of the first drive mechanism 30 may be disposed enclosing the positioning portion 508 of the first trigger mechanism 50. In this case, when the pressing portion 502 is pressed, it is able to trigger the first holding portion 130 to release the moving body 200, and it is able to further provide a driving action for the moving body 200 to move toward the proximal end.

In some examples, the positioning portion 508 of the first trigger mechanism 50 may be of cylindrical shape. The first drive mechanism 30 may be disposed enclosing the positioning portion 508. In some examples, the positioning portion 508 of the first trigger mechanism 50 may be of hollow cylindrical shape. The first drive mechanism 30 may be disposed enclosing the positioning portion 508 or embedded in the positioning portion 508. In some examples, the positioning portion 508 may include two or more arcuate posts, and in the embodiment shown in FIG. 11D, the positioning portion 508 may include an arcuate post 508a and an arcuate post 508b. The first drive mechanism 30 may be embedded between the arcuate post 508a and the arcuate post 508b.

In some examples, an error prevention mechanism 520 may be provided between the first trigger portion 50 and the end portion 120 (see FIG. 7C). In some examples, the error prevention mechanism 520 may be of hairpin shape. In this case, undesired movement of the first trigger mechanism 50 toward the proximal end can be effectively suppressed by the hairpin shaped error prevention mechanism 520. In some examples, the error prevention mechanism 520 may be disposed between the pressing portion 502 of the first trigger mechanism 50 and the first housing 100.

In some examples, a resilient member such as a spring (not shown), may also be provided between the end portion 120 and the first trigger mechanism 50. In some examples, the spring disposed between the end portion 120 and the first trigger mechanism 50 may be in a compressed state. In this case, undesired movement of the first trigger mechanism 50 toward the proximal end is also suppressed by the action of the spring toward the distal end In addition, the spring provided between the end portion 120 and the first trigger mechanism 50 can also facilitate the first trigger mechanism 50 to return to the initial state after the first holding portion 130 is triggered. In this case, the first trigger mechanism 50 can automatically return to the initial state after triggering the first holding portion 130, thereby contributing to providing a reusable applicator 1000.

FIG. 12A is an exploded schematic diagram showing a first perspective of the second housing 140 and the auxiliary mechanism 20; FIG. 12B is an exploded schematic diagram showing a second perspective of the second housing 140 and the auxiliary mechanism 20; FIG. 12C is a schematic cross-sectional diagram showing the auxiliary mechanism 20 mounted to the second housing 140; FIG. 12D is a schematic diagram showing the cover 190 according to an example of the present embodiment.

In some examples, the first bottom portion 210 and the side wall 230 of the moving body 200 may be integrally formed. In other examples, the first bottom portion 210 of the moving body 200 may be detachably assembled with the side wall 230.

In some examples, the first bottom portion 210 may have a joint 214 (see FIG. 12A). In some examples, the number ofjoint(s) 214 may be one or more, such as 1, 2, 3, or 4. In some examples, the side wall 230 may have a moving body restriction portion 238 (described later). The moving body restriction portion 238 may restrict the puncture member 260 disposed within the moving body 200 to suppress undesired rotation of the puncture member 260. In some examples, the moving body restriction portion 238 may be formed as a groove and/or ridge. In some examples, the moving body restriction portion 238 may be two ridges arranged side by side and a groove between the two ridges. The joint 214 is engaged with the side wall 230 by engaging with ends of the two ridges of the moving body restriction portion 238.

In some examples, the second housing 140 may comprise a first limiting mechanism 150 and a second limiting mechanism 170. In some examples, the first limiting mechanism 150 and the second limiting mechanism 170 may communicate with each other. In some examples, the first limiting mechanism 150 and the second limiting mechanism 170 may be of hollow cylindrical structure. In some examples, the first limiting mechanism 150 may releasably hold the moving body 200 and may define a movement path of the moving body 200. In some examples, the second limiting mechanism 170 may define the attachment position for the medical instrument 800.

That is, in the applicator 1000 according to the present embodiment, the housing 10 may comprise a first housing 100 having the first holding portion 130 and a second housing 140 mountable to the first housing 100, the second housing 140 may have the first limiting mechanism 150 and the second limiting mechanism 170 communicated with each other, the second limiting mechanism 170 may be configured to define the attachment position of the medical instrument 800, and the auxiliary mechanism 20 may be mounted to the first limiting mechanism 150 and may move along the first limiting mechanism 150 when the moving body 200 is released by the first holding portion 130. In this case, the movement of the moving body 200 is defined by the first limiting mechanism 150 of the second housing 140, and the attachment position of the medical instrument 800 is defined by the second limiting mechanism 170 of the second housing 140, whereby it is able to facilitate more accurate application of the medical instrument 800 to the host

In some examples, the first limiting mechanism 150 may have a restriction portion 151. The restriction portion 151 may restrict the moving body 200 so as to suppress an undesired rotation of the moving body 200. In some examples, the restriction portion 151 may be a groove and/or a ridge. In some examples, the restriction portion 151 may include a ridge 154 disposed on an inner wall of the first limiting mechanism 150 and extending substantially along the central axis CA. In some examples, the restriction portion 151 may further include a groove 152 disposed on the inner wall of the first limiting mechanism 150 and extending substantially along the central axis CA. In some examples, a length of the ridge 154 and the groove 152 may be less than a height of first limiting mechanism 150, and the ridge 154 and the groove 152 may be substantially collinear in the direction of the central axis CA. In some examples, the restriction portion 151 may further include a notch 156 provided on the first limiting mechanism 150, and the notch 156, the ridge 154, and the groove 152 may be sequentially formed along the same straight line from the proximal end to the distal end. In some examples, the number of restriction portion(s) 151 may be one or more, such as 1, 2, 3, or 4. In some examples, the plurality of restriction portions 151 may be evenly distributed on the side wall of the first limiting mechanism 150.

In some examples, the ridge 154 of the restriction portion 151 may cooperate with a restricted portion 232 (described later) of the moving body 200 including a groove structure, thereby suppressing undesired rotation of the moving body 200. In some examples, the moving body 200 may be mounted to the first limiting mechanism 150 along the groove 152, whereby the groove 152 can provide a guiding function upon mounting. In addition, in some examples, the notch 156 may be used to provide an engagement space for the protrusion 234 (described later) of the moving body 200.

Additionally, in some examples, the first limiting mechanism 150 may also include a reinforcing rib 158 disposed on an inner wall (see FIG. 12B). In some examples, the reinforcing rib 158 may extend substantially in the direction of the central axis CA. In some examples, the number of reinforcing ribs 158 may be multiple, such as 2, 3, 4, 6, etc. In some examples, the reinforcing ribs 158 may be evenly distributed on the inner wall of the first limiting mechanism 150.

Additionally, in some examples, the first limiting mechanism 150 may also include a protrusion 160 (described in detail later) disposed on the inner wall.

In addition, in some examples, the first limiting mechanism 150 may limit the travel of the moving body 200 in a direction along the central axis CA. In this case, the travel of the moving body 200 in the direction along the central axis CA is restricted by the first limiting mechanism 150, whereby the medical instrument 800 received in the receiving portion 250 can be pushed a predetermined distance more accurately, and thus the medical instrument 800 can be applied to the host more accurately.

Additionally, in some examples, the receiving portion 250 may at least partially coincide with the wall of the first limiting mechanism 150, and the end near the distal end of the moving body 200 may at least partially coincide with the wall of the first limiting mechanism 150, as projected in the direction along the central axis CA (see FIG. 12C). In this case, by making the receiving portion 250 and the moving body 200 at least partially coincide with the wall of the first limiting mechanism 150, it is able to effectively limit the travel of the moving body 200 moving along the first limiting mechanism 150 with structural simplification

In addition, in some examples, a protrusion protruding away from the central axis CA in the direction substantially orthogonal to the central axis CA is provided on one end near the distal end of the moving body 200. In this case, excessive travel toward the proximal end of the moving body 200 can be effectively restricted by the protrusion forming a snap with the wall of the first limiting mechanism 150.

Additionally, in some examples, the first limiting mechanism 150 may suppress rotation of the moving body 200. In this case, by suppressing rotation of the moving body 200, undesired rotation of the puncture member 260 during application can thereby be suppressed

Additionally, in some examples, the first limiting mechanism 150 may include a groove and/or a ridge disposed continuously or non-continuously on the inner wall substantially along the direction of the central axis CA. In this case, by engaging the moving body 200 with the groove and/or ridge of the first limiting mechanism 150, it is able to effectively suppress the moving body 200 from undesirably rotating within the first limiting mechanism 150.

In addition, in some examples, the moving body 200 may include a groove and/or a ridge disposed on an outer wall of the side wall 230 substantially in the direction of the central axis CA. In this case, by engaging the groove and/or ridge of the moving body 200 with the groove and/or ridge of the first limiting mechanism 150, it is able to effectively suppress the moving body 200 from undesirably rotating within the first limiting mechanism 150.

In addition, in some examples, the first limiting mechanism 150 and the second limiting mechanism 170 may be of hollow canister shape and an inner diameter of the first limiting mechanism 150 may be no larger than an inner diameter of the second limiting mechanism 170. In this case, by providing the first limiting mechanism 150 and the second limiting mechanism 170 in a hollow canister shape, it is able to facilitate the movement of the auxiliary mechanism 20 along the first limiting mechanism 150 and the second limiting mechanism 170.

Additionally, in some examples, as the moving body 200 is released, the moving body 200 may move along the first limiting mechanism 150 and the receiving portion 250 may move along the second limiting mechanism 170. In this case, by causing the moving body 200 to move along the first limiting mechanism 150 and the receiving portion 250 to move along the second limiting mechanism 170, the medical instrument 800 received in the receiving portion 250 can be more accurately applied to a desired position of the body surface of the host

In some examples, the restricted portion 232 may be provided on a side wall 230 of the moving body 200 (see FIGS. 12A and 12B). The restricted portion 232 may cooperate with the restriction portion 151 of the first limiting mechanism 150 to suppress undesired rotation of the moving body 200. In some examples, the restricted portion 232 may include a groove 235 formed in the outer wall of the side wall 230. In some examples, the groove 235 may be formed between two side-by-side ridges. In some examples, the restricted portion 232 may also include an arm 233 formed by the side wall 230 extending in the direction of the groove 235 toward the distal end. In some examples, the restricted portion 232 may also include a protrusion 234 projecting away from the central axis from the arm 233 in the direction substantially orthogonal to the central axis CA. In some examples, the number of restrictions) 232 may be one or more, such as 1, 2, 3, or 4. In some examples, the number of restricted portions 232 may be the same as the number of restriction portions 151. In some examples, the plurality of restricted portions 232 may be evenly distributed on the side wall 230. In some examples, the arm 233 may be resilient.

In some examples, the projection plane of the protrusion 234 of the restricted portion 232 may be farther from the central axis CA than the projection of the notch 156 of the restriction portion 151, as projected in the direction substantially orthogonal to the central axis CA. In addition, the projection of the ridge 154 and the projection of the groove 235 may substantially engage with each other. Additionally, the projection of the protrusion 234 may substantially engage the projection of the groove 152. In some examples, a length and/or width of the receiving portion 250 may be greater than a diameter of the first limiting mechanism 150.

Hereinafter; with reference to FIG. 12C, action is applied toward the moving body 200 in the direction of the central axis CA, so that the moving body 200 is mounted to the first limiting mechanism 150 via the second limiting mechanism 170. The protrusion 234 may slide into the groove 152, thereby providing a mechanism to guide the mounting. When the protrusion 234 abuts the ridge 154, the ridge 154 compresses the protrusion 234 and elastically deforms the arm 233 so that the protrusion 234 is moved toward the central axis CA and can continue to slide past the ridge 154. As the protrusion 234 projects from the first limiting mechanism 150 in the direction of the central axis CA toward the distal end, the arm 233 returns to an initial state such that the protrusion 234 projects from the first limiting mechanism 150 in the direction substantially orthogonal to the central axis CA.

The moving body 200 is mounted to and combined with the first limiting mechanism 150, and the groove 235 of the moving body 200 can be engaged with the ridge 154 of the first limiting mechanism 150. Thus, when the moving body 200 moves along the first limiting mechanism 150, the moving body 200 can be limited in the direction in which the ridge portion 154 extends. Additionally, engagement of the groove 235 with the ridge 154 can also provide a mechanism to suppress rotation to suppress undesired rotation of the moving body 200 within the first limiting mechanism 150. In this case, when the puncture mechanism 260 penetrates the subcutaneous skin of the host as the main body 200 is moved, since the undesired rotation is effectively suppressed, the discomfort given to the host during application can be reduced.

In addition, as described above, projection is made in the direction along the central axis CA, the projection of the protrusion 234 is further from the central axis CA than the projection of the notch 156. In this case, as the moving body 200 is mounted to and engaged with the first limiting mechanism 150, the amount of displacement of the moving body 200 toward the proximal end can be restricted by the protrusion 234 forming a snap connection with the notch 156.

In addition, as described above, a length and/or width of the receiving portion 250 connected to the second bottom portion 220 of the moving body 200 is greater than a diameter of the first limiting mechanism 150 in the direction substantially orthogonal to the central axis CA. In this case, when the moving body 200 is mounted to and engaged with the first limiting mechanism 150, the amount of displacement that the moving body 200 travels toward the distal end can be restricted by the receiving portion 250.

In some examples, the second limiting mechanism 170 may include an upper bottom portion 172, a lower bottom portion 176, and a side portion 174 connecting the upper bottom portion 172 and the lower bottom portion 176 (see FIG. 12B). In some examples, the upper bottom portion 172 may be formed in a manner such that an end near the proximal end of the first limiting mechanism 150 extends outwardly in the direction substantially orthogonal to the central axis CA. The side portion 174 may be of hollow canister structure, and the direction in which the side portion 174 extends may be substantially parallel to the central axis CA. The lower bottom portion 176 may be formed in such a manner that an end near the proximal end of the side portion 174 extends outwardly in the direction substantially orthogonal to the central axis CA.

In some examples, a width of the lower bottom portion 176 protruding from the side portion 174 in the direction substantially orthogonal to the central axis CA may be substantially equal to a thickness of the peripheral portion 110 of the first housing 100.

In some examples, the second limiting mechanism 170 may comprise a rib groove 178 disposed on the side portion 174 (see FIG. 12B). In some examples, the number of rib groove(s) 178 may be one or more, such as 1, 2, 3, or 4. In some examples, the rib groove 178 may be formed in such a manner that two ridges extending substantially in the direction of the central axis CA are disposed side by side on the side portion 174, and the rib groove 178 may be formed between the two side-by-side ridges.

In some examples, the rib groove 178 may cooperate with the peripheral rib 112 of the peripheral portion 110 to make the first housing 100 fitted with the second housing 140. In some examples, the number of the rib grooves 178 may be the same as the number of the peripheral ribs 112. In some examples, the peripheral rib 112 may fit into the rib groove 178 in a plug fashion substantially along the direction of the central axis CA. In some examples, an inner contour of the peripheral portion 110 is substantially the same as an outer contour of the second limiting mechanism 170. As the first housing 100 and the second housing 140 are moved toward each other in a direction substantially along the central axis CA, the peripheral rib 112 may enter the rib groove 178, thereby mounting the first housing 100 to the second housing 140.

In some examples, the second limiting mechanism 170 may also include a fitting hole 180 provided on the upper bottom portion 172 (see FIG. 12B). In some examples, the number of the fitting hole(s) 180 may be one or more, such as 1, 2, 3, or 4. In some examples, the number of the fitting holes 180 may be the same as the number of the connection columns 114 of the first housing 100. The fitting hole 180 may be fitted with the connection column 114 to fit and engage the first housing 100 to the second housing 140. The attachment post 114 may abut the fitting hole 180.

In some examples, the second limiting mechanism 170 may also include a flange 183 (see FIG. 12B). In some examples, a hollow portion 186 may also be provided on the upper bottom portion 172 of the second limiting mechanism 170 (see FIG. 12B). In this case, when the applicator 1000 is fitted and engaged with the cartridge device 2000, inside and outside air pressure is balanced by the hollow portion 186 so that the applicator 1000 is engaged with the cartridge device 2000.

In some examples, the receiving portion 250 may move along the second limiting mechanism 170. In some examples, the attachment position of the medical instrument 800 can be defined by attaching the second limiting mechanism 170 to the body surface of the host when the applicator 1000 is operated.

In some examples, the second limiting mechanism 170 may also include a magnet 184 disposed on the upper bottom portion 172 (see FIG. 12B). In some examples, the second limiting mechanism 170 may also include a magnet securing portion 185 disposed on the upper bottom portion 172, which may be used to secure the magnet 184. In some examples, the magnet securing portion 185 may be two or more columnar protrusions along the direction of the central axis CA. The magnet 184 may be secured between a plurality of cylindrical protrusions. In this case, the magnet 184 disposed on the second limiting mechanism 170 may cooperate with the switch module 884 of the medical instrument 800 to control the opening of the switch module 884. Specifically, as the moving body 200 is held, the medical instrument 800 accommodated in the receiving portion 250 is close to the magnet 184 on the second limiting mechanism 170, and the switch module 884 is maintained in a non-conductive state by the magnetic action of the magnet 184. As the moving body 200 is released, the moving body 200 moves toward the proximal end and applies the medical instrument 800 accommodated in the receiving portion 250 to the host, the medical instrument 800 is distant from the magnet 184 on the second limiting mechanism 170, and the switch module 884 is no longer subjected to the magnetic action of the magnet 184 and is switched into a conductive state.

Additionally, in some examples, a side clamping groove 182 (see FIG. 12A) is provided on an inner wall of the side portion 174 of the second limiting mechanism 170. In some examples, the number of the side clamping groove(s) 182 may be one or more, such as 1, 2, 3, or 4. In some examples, the plurality of side clamping grooves 182 may be evenly distributed on the inner wall of the side portion 174. In some examples, the side clamping groove 182 may extend substantially along the direction of the central axis CA. The side clamping groove 182 may cooperate with a cover clamping portion 194 (described later) of the cover 190 to make the second housing 140 fitted and engaged with the cover 190.

In some examples, a first type rib 187 is provided on the inner wall of the side portion 174 of the second limiting mechanism 170. The first type rib 187 may serve as a guide (described later) when the applicator 1000 cooperates with the cartridge device 2000. In some examples, the number of the first type rib(s) 187 may be multiple, such as 1, 2, 3, or 4.

In some examples, a second type rib 188 is provided on the inner wall of the side portion 174 of the second limiting mechanism 170. The second type rib 188 may act to pressurize (described later) when the applicator 1000 cooperates with the cartridge device 2000. In some examples, the number of the second type rib(s) 188 may be one or more, such as 1, 2, 3, or 4.

In some examples, a third type nb 189 is provided on the inner wall of the side portion 174 of the second limiting mechanism 170. The third type rib 189 may act as a bias (described later) when the applicator 1000 cooperates with the cartridge device 2000. In some examples, the number of the third type rib(s) 189 may be one or more, such as 1, 2, 3, or 4.

In some examples, the housing 10 may further include a cover 190 detachably covering the second housing 140 (see FIG. 7C).

In some examples, the cover 190 may include a cover bottom portion 192 and a cover clamping portion 194 disposed on the cover bottom portion 192 (see FIG. 12D). The cover clamping portion 194 may cooperate with the side clamping groove 182 of the second housing 140 to mount the cover 190 to the second housing 140. The number of the cover clamping portion(s) 194 may be one or more, such as 1, 2, 3, or 4. The number of the cover clamping portion(s) 194 may be equal to the number of the side clamping groove(s) 182. In the embodiment shown in FIG. 12D, the cover clamping portions 194 may include a cover clamping portion 194a and a cover clamping portion 194b. The cover clamping portion 194a and the cover clamping portion 194b may be distributed on opposite sides of the cover bottom portion 192 along an axis of symmetry of the cover 190.

In some examples, the cover clamping portion 194 may include an arm extending from the cover bottom portion 192 substantially along the central axis CA toward the distal end and a protrusion protruding from an end of the arm in a direction away from the central axis CA. The arm of the cover clamping portion 194 may be resilient. As the cover 190 is fitted and engaged with the second housing 140, the protrusion of the cover clamping portion 194 can fit into the side clamping groove 182 of the second housing 140.

In some examples, the cover 190 may also include a cover pillar 196 (see FIG. 12D). An axis of the cover pillar 196 may be substantially parallel to the central axis CA. In some examples, the cover pillar 196 may be formed by extending from cover bottom portion 192 along central axis CA toward the distal end In some examples, when the moving body 200 is held, a vertical distance between the surface facing the proximal end of the medical instrument 800 accommodated within the receiving portion 250 and the lower bottom portion 176 of the second housing 140 may be approximately equal to a height of the cover pillar 196. In this case, when the cover 190 is fitted and engaged with the second housing 140, the medical instrument 800 accommodated in the receiving portion 250 is supported by the cover pillar 196, whereby it is able to further suppress the moving body 200 from being undesirably released due to, for example, a mis-operation

In some examples, the cover 190 may also include a flange 198 (see FIG. 12D). The location of the flange 198 of the cover 190 may correspond to the flange 183 of the second housing 140. Specifically, when the cover 190 is fittingly engaged with the second housing 140, projected in the direction along the central axis CA, a projection plane of the flange 198 and a projection plane of the flange 183 may substantially coincide, or the projection plane of the flange 198 may substantially cover the projection plane of the flange 183.

FIG. 13A is an exploded schematic diagram showing a first perspective of the moving body 200 and the puncture member 260 according to an example of the present embodiment; FIG. 13B is an exploded schematic diagram showing a second perspective diagram of the moving body 200 and the puncture member 260 according to an example of the present embodiment; FIG. 13C is a schematic cross-sectional diagram showing the puncture member 260 according to an example of the present embodiment when held; FIG. 13D is a schematic cross-sectional diagram showing the puncture member 260 according to an example of the present embodiment when released; FIG. 13E is a schematic cross-sectional diagram illustrating pre-fitting of the moving body 200 and the puncture member 260 according to an example of the present embodiment.

In some examples, the moving body 200 and the puncture member 260 may be integrally fixedly connected. In other examples, the moving body 200 and the puncture member 260 may be detachably fitted and engaged In some examples, the puncture member 260 may be releasably held by the moving body 200.

In some examples, the moving body 200 may have a first bottom portion 210 near the distal end, a second bottom portion 220 near the proximal end, and a side wall 230 connecting the first bottom portion 210 and the second bottom portion 220. In some examples, a hollow portion may be formed between the first bottom portion 210, the second bottom portion 220, and the side wall 230. In some examples, the receiving portion 250 may be disposed on the second bottom portion 220 and facing the proximal end In some examples, the puncture member 260 may be disposed within the hollow portion. In some examples, the second bottom portion 220 may have a through hole 226. Additionally, in some examples, the puncture member 260 may pass through the through-hole 226.

In some examples, the puncture member 260 may be releasably disposed within the hollow portion. In addition, the puncture member 260 may be configured to move within the hollow portion relative to the moving body 200 when released. In some examples, the second drive mechanism 40 may be provided between the second bottom portion 220 of the moving body 200 and the puncture member 260. The second drive mechanism 40 is configured to act on the puncture member 260 toward the distal end When the puncture member 260 is released, the second drive mechanism 40 may act on the puncture member 260 toward the distal end to move the puncture member 260 away from the host. In some examples, the second drive mechanism 40 may be disposed between the bearing 280 of the puncture member 260 and the second bottom portion 220 of the moving body 200. As a result, it can be facilitated that the second drive mechanism 40 acts on the puncture member 260 away from the receiving portion 250.

In some examples, as described above, the moving body 200 may include a side wall 230 (see FIG. 13A and FIG. 13B). In some examples, the moving body 200 may include a restriction portion 238 disposed on the side wall 230. The puncture member 260 may have a restricted portion 285 (described later). When the puncture member 260 is fitted with the moving body 200, the moving body 200 may impose a restriction on the restricted portion 285 by the restriction portion 238 to restrict movement of the puncture member 260. In some examples, the number of the restriction portion(s) 238 may be one or more, such as 1, 2, 3, or 4. In some examples, the plurality of restrictions 238 may be evenly distributed on the inner wall of the side wall 230.

In some examples, the moving body 200 may further comprise a second holding portion 242 (see FIG. 13A and FIG. 13B). The puncture member 260 may have a second locking portion 286. When the puncture member 260 is fitted with the moving body 200, the moving body 200 may be interlocked with the puncture member 260 through the second holding portion 242 to hold the puncture member 260. In some examples, the holding of the puncture member 260 by the second holding portion 242 may be releasable. In some examples, the number of second holding portion(s) 242 may be one or more, such as 1, 2, 3, or 4. In one example, the plurality of second holding portions 242 may be evenly distributed on the side wall 230.

Additionally, in some examples, the moving body 200 may be configured to suppress rotation of the puncture member 260. In this case, the user experience during application of the medical instrument 800 can be improved by suppressing undesired rotation of the puncture member 260 during application. In some examples, the moving body 200 may suppress undesired rotation of the puncture member 260 during application via the restriction portion 238.

In addition, in some examples, the moving body 200 may include a groove and/or a ridge disposed on the inner wall of the side wall 230 substantially in the direction of the central axis CA. In this case, undesired rotation of the puncture member 260 during application can be effectively suppressed by the groove and/or ridge provided on the inner wall of the side wall of the moving body 200 cooperating with the groove and/or ridge of the puncture member 260. That is, in some examples, the restriction portion 238 of the moving body 200 may be a groove and/or ridge provided on the inner wall of the side wall 230 substantially in the direction of the central axis CA.

In some examples, the restriction portion 238 of the moving body 200 may be formed as a groove structure (see FIG. 13A and FIG. 13B). In some examples, the restriction portion 238 may include two ridges disposed side by side on the inner wall of the side wall 230 substantially along the direction of the central axis CA and a groove formed between the two ridges.

In addition, in some examples, the moving body 200 may have a cutout 242 substantially along the direction of the central axis CA. In this case, by providing the cutout 242 in the direction of the central axis CA on the moving body 200, it is able to facilitate the action on the puncture member 260 provided in the hollow portion of the moving body 200.

In addition, in some examples, a protrusion 160 that may protrude toward the central axis CA via the cutout 242 may be provided on the inner wall of the first limiting mechanism 150. In this case, when the moving body 200 moves along the first limiting mechanism 150, the protrusion 160 provided on the inner wall of the first limiting mechanism 150 can act on the puncture member 260 via the cutout 242 of the moving body 200, thereby providing a trigger mechanism for the puncture member 260 with a simplified structure.

In some examples, the second holding portion 242 of the moving body 200 may be a cutout formed on the side wall 230 (see FIG. 13A and FIG. 13B). In some examples, the second holding portion 242 may be a first cutout 244 and a second cutout 246 extending substantially along the central axis CA and communicating with each other. The first cutout 244 may be near the proximal end and the second cutout 246 may be near the distal end In some examples, a width of the first cutout 244 and a width of the second cutout 246 may be different. In some examples, the width of the first cutout 244 may be greater than the width of the second cutout 246.

In some examples, the receiving portion 250 may have a restraining portion 252 for restraining the attachment portion 860. In this case, the attachment portion 860 of the medical instrument 800 accommodated in the receiving portion 250 is restrained by the restraining portion 252, whereby the medical instrument 800 can be accommodated more stably.

In some examples, the restraining portion 252 of the receiving portion 250 may include an arm 254 and a protruding portion 256. Wherein the arm 254 may extend outwardly from the bottom portion of the receiving portion 250 in substantially the same direction as the side portion In some examples, the arm 254 and the side portion of the receiving portion 250 may be located approximately in a same circumferential direction In some examples, the arm 254 may be resilient In some examples, the arm 254 may converge toward the central axis CA. In some examples, the protruding portion 256 may be a protrusion disposed on an end of the arm 254. In some examples, the protruding portion 256 may protrude toward the central axis CA. In this case, the restraining portion 252 can clamp the medical instrument 800 accommodated in the receiving portion 250, thereby more stably accommodating the medical instrument 800.

In some examples, the number of the restraining portion(s) 252 may be one or more, such as 1, 2, 3, or 4. In some examples, the restraining portions 252 may be evenly distributed along the side portion of the receiving portion 250.

In some examples, the puncture member 260 may include a sharp object 270 and a holder 280 that supports the sharp object 270 (see FIG. 13A and FIG. 13B). In some examples, the holder 280 of the puncture member 260 may be disposed within the hollow portion of the moving body 200, the receiving portion 250 may be disposed at the second bottom portion 220, the second bottom portion 220 may have a through hole 226, and the sharp object 270 may pass through the through hole 226 of the second bottom portion 220. In this case, the sharp object 270 can be engaged with the medical instrument 800 accommodated in the receiving portion 250 by disposing the sharp object 270 through the through hole 226 of the second bottom portion 220, thereby enabling the medical instrument 800 to be at least partially placed subcutaneously in the host through the puncture member 260.

Additionally, in some examples, the moving body 200 may also include a structure 224 disposed on the second bottom portion 220 for positioning the second drive mechanism 40 (see FIG. 13C). In some examples, the structure 224 may be of columnar shape. In some examples 224 may be of hollow cylindrical shape. In this case, the second drive mechanism 40 may be positioned by being disposed enclosing the structure 224, thereby enabling to define the driving position of the second drive mechanism 40.

Additionally, in some examples, a spacing of the holder 280 from the first bottom portion 210 may be no less than a length of the sharp object 270 protruding from the receiving portion 250 when the puncture member 260 is held In this case, it is able to provide a moving space for the puncture member 260 to move away from the host by setting the distance between the holder 280 and the first bottom portion 210 to be not less than the length of the sharp object 270 protruding from the receiving portion 250, thereby capable of reducing an undesired injury by the puncture member 260 to the host.

In some examples, the sharp object 270 and the holder 280 may be integrally formed In other examples, the sharp object 270 and the holder 280 may be configured to be detachably fitted together. Thus, additional sterilization of the sharp object 270 can be facilitated.

In some examples, the restricted portion 285 of the puncture member 260 may be formed as a ridge structure (see FIG. 13B). In some examples, the restricted portion 285 may include two grooves disposed side by side on the side wall of the holder 280 substantially along the direction of the central axis CA and a ridge formed between the two grooves. In some examples, the number of the restricted portion(s) 285 may be one or more, such as 1, 2, 3, or 4. In some examples, the number of the restricted portions 285 and the number of restriction portions 238 may be equal.

Additionally, in some examples, the holder 280 may include a groove and/or a ridge disposed on an outer wall substantially in the direction of central axis CA. In this case, the undesired rotation of the puncture member 260 can be effectively suppressed by the cooperation of the groove and/or ridge of the holder 280 with the groove and/or ridge on the inner wall of the side wall of the moving body 200. That is, the restricted portion 285 of the puncture member 260 may be formed as a groove and/or ridge disposed on the outer wall substantially in the direction of the central axis CA. The restricted portion 285 of the puncture member 260 may cooperate with the restriction portion 238 of the moving body 200 to suppress undesired rotation of the puncture member 260.

Additionally, in some examples, the puncture member 260 may include a second locking portion 286 configured to releasably interlock with the second holding portion 242. Thus, the puncture member 260 can be releasably held by the second holding portion 242 through cooperation of the second locking portion 286 with the second holding portion 242. In some examples, the second locking portion 286 may be disposed on the holder 280 of the puncture member 260.

In some examples, the second locking portion 286 may include an arm 287 extending substantially along the direction of the central axis CA and a protrusion 288 cooperating with the arm 287 and projecting away from the central axis CA substantially along the direction orthogonal to the central axis CA. In this case, when the puncture member 260 is placed in the hollow portion of the moving body 200, the second locking portion 286 overlaps the cutout 242 of the moving body 200, whereby the puncture member 260 can be held with a simplified structure. In some examples, the arm 287 of the second locking portion 286 may be resilient in the direction substantially orthogonal to the central axis CA of the applicator 1000.

That is, in some examples, the moving body 200 may have a cutout 242 substantially along the direction of the central axis CA. In some examples, the protrusion 288 of the second locking portion 286 may pass through the cutout 242 when the puncture member 260 is held. In this case, by overlapping the protrusion 288 of the second locking portion 286 with the cutout 242 of the moving body 200, the second locking portion 286 can be held with a simplified structure.

Additionally, in some examples, when the puncture member 260 is released, the arm 288 of the second locking portion 286 may be pressed toward the central axis CA. Thus, the second locking portion 286 can be released by easy operation

Additionally, in some examples, the second holding portion 242 and the second locking portion 286 may be releasably interlocked by at least one structure of a buckle, a hook, a latch, or a pin. In this case, the second holding portion 242 and the second locking portion 286 are releasably interlocked by at least one structure of a buckle, a hook, a latch, or a pin, thereby providing a releasable interlock.

In some examples, the second locking portion 286 of the puncture member 260 may be formed as a shoulder structure (see FIG. 13A and FIG. 13B). In some examples, the second locking portion 286 may include an arm 287 extending substantially in the direction of the central axis CA toward the distal end, and a protrusion 288 projecting from the end of the arm 287 in the direction substantially orthogonal to the central axis CA away from the central axis CA. In some examples, the arm 287 may be resilient In some examples, along the central axis CA and in the direction toward the distal end, the arm 287 may gradually far away from the central axis CA. In some examples, projected in the direction of the central axis CA, the projection plane of the protrusion 288 and the projection plane of the first cutout 244 may coincide in whole or in part. In some examples, the number of the second locking portion(s) 286 may be one or more, such as 1, 2, 3, or 4. In some examples, the number of the second locking portions 286 and the number of the second holding portions 242 may be equal.

Additionally, in some examples, a width of the surface, facing the distal end, of the protrusion 288 may be less than a width of the first cutout 244 and may be greater than a width of the second cutout 246.

In some examples, when the first limiting mechanism 150 and the moving body 200 are assembled, the protrusion 160 provided on the inner wall of the first limiting mechanism 150 may sequentially pass through the second cutout 246 and the first cutout 244. In this case, by providing the first cutout 244, a holding mechanism for the puncture member 260 can be provided, and in addition, by providing the second cutout 246, a structure that facilitates assembly can be provided. The protrusion 160 passes through the second cutout 246 and enters the first cutout 244, and the protrusion 288 of the second locking portion 286 protrudes through the first cutout 244, thereby providing a holding mechanism for the puncture member 260, and the protrusion 160 can pass through the first cutout 244 and abut the protrusion 288 of the second locking portion 286, thereby providing a trigger mechanism for triggering the second locking portion 286 to be released through the protrusion 160 and the first cutout 244.

With reference to FIG. 13E, the restricted portion 285 of the puncture member 260 may be snapped into the restriction portion 238 (see FIG. 13E) by acting on the puncture member 260 toward the proximal end so that the puncture member 260 enters the hollow portion of the moving body 200 and the restricted portions 285 of the puncture member 260 are aligned with the restriction portions 238 of the moving body 200 one by one. Thus, on the one hand, it is able to provide guidance for the fitting of the puncture member 260 with the moving body 200, on the other hand, the puncture member 260 fitted in the moving body 200 is defined by the restriction portion 238, and undesired rotation can be effectively suppressed.

In addition, referring to FIG. 13C and FIG. 13D, when the protrusion 288 of the second locking portion 286 of the puncture member 260 is moved to the first cutout 244, since it is no longer pressed by the inner wall of the side wall 230, the arm 287 returns to its original state, i.e. the projection plane of the protrusion 288 is overlapped with the projection plane of the first cutout 244 in whole or in part along the central axis CA, and since a width of the surface facing the distal end of the protrusion 288 is greater than a width of the second cutout 246, the protrusion 288 is placed in the first cutout 244 and the surface facing the distal end of the protrusion 288 abuts against the intersection of the first cutout 244 and the second cutout 246 (see FIG. 13C), thereby being held in the hollow portion of the moving body 200. When the arm 287 is acted on to pivot in the direction of the central axis CA, the protrusion 288 moves in the direction of the central axis CA, i.e. converges inwardly. In this case, the surface facing the distal end of the protrusion 288 no longer abuts the intersection of the first cutout 244 and the second cutout 246, thereby allowing the puncture member 260 to be released by the moving body 200 (see FIG. 13D).

In some examples, when the puncture member 260 is released by the moving body 200, the second drive mechanism 40 drives the puncture member 260 toward the distal end to move the puncture member 260 away from the host.

In some examples, the second drive mechanism 40 may have the same or similar configuration as the first drive mechanism 30, the characterization of the first drive mechanism 30 can be referred to for the characterization of the second drive mechanism 40. In some examples, the second drive mechanism 40 may be a spring. Thus, the actuation mechanism can be provided for the puncture member 260 through a simplified structural design

In some examples, the second drive mechanism 40 may be disposed between the holder 280 and the second bottom portion 220, and when the puncture member 260 is held, the holder 280 is spaced from the first bottom portion 210 no less than a depth to which the sensor 820 is implanted subcutaneously. In this case, by providing a sufficient amount of moving space for the puncture member 260, it is thereby able to facilitate the removal of the puncture member 260 from the host.

In some examples, the puncture member 260 may have a positioning portion 289 disposed on the holder 280 (see FIG. 13C or FIG. 13D). In some examples, the positioning portion 289 may be of a columnar structure. One end of the second drive mechanism 40 may be disposed enclosing the positioning portion 289 of the puncture member 260, and the other end of the second drive mechanism 40 may be disposed enclosing the positioning portion 212 of the moving body 200. In this manner, the second drive mechanism 40 is disposed between the moving body 200 and the puncture member 260.

In some examples, the applicator 1000 also provides a trigger mechanism to de-interlock the second holding portion 242 from the second locking portion 286, thereby releasing the puncture member 260 from the moving body 200. That is, in some examples, the applicator 1000 further includes a second trigger mechanism configured to detach the second holding portion 242 from the second locking portion 286 to release the puncture member 260. Thus, the puncture member 260 can be conveniently released by the second trigger mechanism.

As described above, the second locking portion 286 is formed as a shoulder structure capable of abutting the second holding portion 242, and is held by the second holding portion 242 in a clamp manner. In some examples, a trigger mechanism is provided that is capable of actuating the second holding portion 242 or the second locking portion 286 to detach the second holding portion 242 from the second locking portion 286.

In some examples, a trigger mechanism acting on the second holding portion 242 and the second locking portion 286 may be disposed on the travel path of the second locking portion 286. The trigger mechanism may be actuated as the moving body 200 moves toward the proximal end In some examples, the trigger mechanism may be a protrusion disposed on the travel path of the second locking portion 286. In this case, when the second locking portion 286 passes through the protrusion, the arm 287 of the second locking portion 286 may be pressed toward the central axis CA and elastically deformed to separate the protrusion 288 of the second locking portion 286 from the first cutout 244 and the second cutout 246.

In some examples, as previously described, the protrusion 160 is provided on the inner wall of the first limiting mechanism 150 (see FIG. 12B). As the moving body 200 moves along the first limiting mechanism 150, the first cutout 244 and the second cutout 246 of the moving body 200 may pass through the protrusion. In addition, when the puncture member 260 is held in the hollow portion of the moving body 200, the second locking portion 286 of the puncture member 260 is deformed by being pressed by the protrusion 160 and leaves the intersection of the first cutout 244 and the second cutout 246, so that the puncture member 260 is released.

In addition, when the moving body 200 and the first limiting mechanism 150 are assembled, the protrusion 160 may pass through the second cutout 246 and the first cutout 244 in sequence until the moving body 200 is fitted and engaged with the first limiting mechanism 150. By means of the protrusion 160 provided on the inner wall of the first limiting mechanism 150 and the first cutout 244 and the second cutout 246 provided on the side wall 230 of the moving body 200, it is able to provide an in travelling trigger mechanism without any adverse impact on the assembling of the moving body 200 with the first limiting mechanism 150.

Now, the application process of the applicator 1000 will be described again: the process applying the medical instrument 800 to the host with the applicator 1000 may include an application phase, as well as a retraction phase. During the application phase, the medical instrument 800 may be applied to the host, while during the retraction phase, the puncture member 260 may quit the host.

In the application phase, the actuation portion 504 of the first trigger mechanism 50 is enabled to move toward the proximal end by pressing the pressing portion 502 of the first trigger mechanism 50; then, when the actuation portion 504 moves to the first holding mechanism 130, the actuation portion 504 actuates the first holding mechanism 130 to pivot in the direction away from the central axis CA (see FIG. 10I and FIG. 10J) to separate the first holding portion 130 from the first locking portion 236 (see FIG. 9B and FIG. 9C), so that the moving body 200 is released; then, the first drive mechanism 30 acts on the moving body 200 toward the proximal end to move the moving body 200 toward the proximal end; Then, the medical instrument 800 accommodated within the receiving portion 250 is then placed at least partially subcutaneously in the host under the action of the puncture member 260.

In some examples, the applicator 1000 may also not include the first drive mechanism 30. In this case, after released, the moving body 200 may also be driven toward the host by means of human power; so that the medical instrument 800 accommodated in the receiving portion 250 is applied to the host through the puncture member 260.

In the retraction phase, as the moving body 200 moves toward the proximal end along the first limiting mechanism 150, the protrusion 160 provided on the inner wall of the first limiting mechanism 150 and the second locking portion 286 of the puncture member 260 approach to each other through the first cutout 244 provided on the moving body 200, and the protrusion 160 presses the second locking portion 286 to pivot the second locking portion 286 in a direction close to the central axis CAto separate the second locking portion 286 from the second holding portion 242 (see FIG. 13C and FIG. 13D); Then, the second drive mechanism 40 acts on the puncture member 260 toward the distal end to move the puncture member 260 toward the distal end and quit the host.

In some examples, the puncture member 260 and the moving body 200 may also be fixedly connected. Additionally, in some examples, the applicator 1000 may not include the second drive mechanism 40. In this case, when the medical instrument 800 is applied to the host, the moving body 200 may be integrally driven in a direction facing away from the host by means of human power; whereby the puncture member 260 fixedly connected to the moving body 200 may follow the moving body 200 to quit the host.

In addition, it should be noted that, during the retraction phase, the first drive mechanism 30 (or human force) may still exert an action on the moving body 200 toward the host In other words, for the first drive mechanism 30 configured as an elastic member (e.g. a spring), when the moving body 200 is driven toward the host and the medical instrument 800 accommodated in the receiving portion 250 is attached to the body surface of the host, the first drive mechanism 30 may still be in the energy storage state. In this case, the medical instrument 800 is closely attached to the body surface of the host under the action of the first drive mechanism 30 in the retraction stage, thereby effectively reducing the possibility of the medical instrument 800 being detached from the host in the retraction stage.

FIG. 14A is an exploded schematic diagram showing the puncture member 260 according to an example of the present embodiment; FIG. 14B is a assembly schematic diagram showing the puncture member 260 according to an example of the present embodiment; FIG. 14C is an enlarged schematic diagram showing a needle shape portion 272 according to an example of the present embodiment; FIG. 14D is a schematic diagram showing the assembly of the sharp object 270 and the sensor 820 according to an example of the present embodiment.

As described above, in the present embodiment, the puncture member 260 may include a sharp object 270 and a holder 280 that supports the sharp object 270 (see FIG. 14A). In some examples, the sharp object 270 and the holder 280 may be integrally formed. In other examples, the sharp object 270 may be detachably fitted with and engaged to the holder 280. In some examples, the sharp object 270 may have a groove and the medical instrument 800 may be fully or partially disposed in the groove. In some examples, the second drive mechanism 40 may act on the holder 280. In this case, by placing the medical instrument 800 wholly or partially within the channel of the sharp object 270, it is thereby able to facilitate at least partial placement of the medical instrument 800 subcutaneously in the host through the puncture member 260. In some examples, the implant portion of the sensor 820 may be placed within the groove 274 of the sharp object 270.

In some examples, the sharp object 270 may include a needle shape portion 272 (see FIG. 14A). In some examples, a groove 274 extending along a length of the needle shape portion 272 may be provided on the needle shape portion 272. The medical instrument 800 may be at least partially disposed within the groove 274 of the sharp object 270. In this case, the sensor 820 may be received in the groove 274 so that the sensor 820 is positioned subcutaneously in the host following the sharp object 270.

In some examples, the sharp object 270 may include a cap portion 276 connected with the needle shape portion 272 (see FIG. 14A). In some examples, the needle shape portion 272 may be mounted to the holder 280 through the cap portion 276. In some examples, the cap portion 276 may include a first portion 277, a second portion 278, and a third portion 279 connected in series. In some examples, the first portion 277, the second portion 278, and the third portion 279 may be three columnar structures having different diameters. Additionally, in some examples, a diameter of the second portion 278 may be less than a diameter of the first portion 277 and less than a diameter of the third portion 279, thereby forming an annular recess.

In some examples, the holder 280 may include a sharp object fixing portion 282 (see FIG. 14A). In some examples, the number of sharp object fixing portions 282 may be multiple, such as 2, 3, or 4. Additionally, a plurality of sharp object fixing portions 282 may be disposed circumferentially about the central axis CAto clamp the cap portion 276. In some examples, the holder 280 may have a plurality of finger shape portions that converge toward one another, and the cap portion 276 may be releasably held by the plurality of finger shape portions. That is, the sharp object fixing portion 282 of the holder 280 may be formed as a plurality of finger shape portions converging toward one another. In this case, assembly and disassembling of the sharp object 270 and the holder 280 can be facilitated by the cooperation of the cap portion 276 of the sharp object 270 with the plurality of finger shape portions of the holder 280.

In some examples, the sharp object fixing portion 282 may gradually converge toward the central axis CAalong the central axis CA toward the proximal end Thus, a mechanism for clamping the cap portion 276 can be formed. In some examples, the sharp object fixing portion 282 may include an arm 283 extending substantially in the direction of the central axis CA and a protrusion 284 (see FIG. 14A) protruding outwardly from an end of the arm 283 toward the central axis CA. In some examples, the arm 283 may be resilient. When the sharp object 270 is fitted with the holder 280, the protrusion 284 of the sharp object fixing portion 282 may be embedded into the annular recess of the cap portion 276. The first portion 277, the third portion 279, and the annular recess cooperate to secure the sharp object 270. The first portion 277 and the third portion 279 abut against the protrusion 284 from two directions, respectively. Undesirable movement of the sharp object 270 relative to the holder 280 along the central axis CA can thereby be suppressed

In some examples, the sharp object 270 of the puncture member 260 may be fitted with the sensor 820. Specifically with reference to FIG. 14D, in some examples, the needle shape portion 272 of the sharp object 270 may be placed within the sensor engagement hole 842 on the connection portion 840, and the implant portion and the extending portion of the sensor 820 may be disposed within the groove 274 of the sharp object 270.

FIG. 15A is a schematic perspective diagram showing a cartridge device 2000 according to an example of the present embodiment; FIG. 15B is a schematic top diagram showing a cartridge body 2100, a mounting table 2200, and a fence structure 2400 of the cartridge device 2000 according to an example of the present embodiment; FIG. 15C is a schematic top diagram showing a platform 2300 of the cartridge device 2000 according to an example of the present embodiment.

In some examples, the medical instrument 800 may be fully or partially packaged within the cartridge device 2000. In some examples, the sensor 820 of the medical instrument 800 may be packaged within the cartridge device 2000. In other examples, the sensor 820 and the connection portion 840 of the medical instrument 800 may be packaged together within the cartridge device 2000. In other examples, the sensor 820, the connection portion 840, and the sharp object 270 of the puncture member 260 may be packaged together within the cartridge device 2000.

In some examples, the cartridge device 2000 may include a cartridge body 2100 and a mounting table 2200 disposed inside the cartridge body 2100 (see FIG. 15A). The mounting table 2200 may be used to mount a portion of the medical instrument 800, such as the sensor 820 and the connection portion 840 of the medical instrument 800. In some examples, the mounting table 2200 may be formed as a columnar protrusion extending upward from a bottom portion of the cartridge body 2100. In some examples, the central axis of the mounting table 2200 and the central axis of the cartridge body 2100 may be substantially parallel.

In some examples, the cartridge body 2100 may include a concave edge 2110 formed on the inner wall (see FIG. 15A). In this case, when the applicator 1000 cooperates with the cartridge device 2000 (to be described later), the concave edge 2110 on the cartridge body 2100 cooperates with the flange 183 of the second housing 140, thereby being able to facilitate to provide guidance when the applicator 1000 is combined with the cartridge device 2000.

In some examples, a top profile of the mounting table 2200 and a top profile of the connection portion 840 may be substantially the same, thereby facilitating the mounting of the connection portion 840. In some examples, the mounting table 2200 may have a hollow portion 2210 (see FIG. 15B). Thus, when the connection portion 840 to which the sensor 820 is connected is mounted on the mounting table 2200, a portion protruding from the connection portion 840 of the sensor 820 may be disposed in the hollow portion 2210. In addition, the central axis of the hollow portion 2210 may be substantially parallel to the central axis of the mounting table 2200.

In some examples, the mounting table 2200 may also include a fixed portion 2220 disposed at a top corner (see FIG. 15B). In this case, the fixed portion 2220 is combined with a concave portion at the top corner of the connection portion 840, whereby the connection portion 840 can be more stably mounted. With reference to FIG. 3, the connection portion 840 may also include a recess or notch provided at the top corner.

In some examples, the mounting table 2200 may also include a storage space 2230 (see FIG. 15B). In some examples, the storage space 2230 may be formed by providing a groove on the mounting table 2200 along the axis of the mounting table 2200. In some examples, a desiccant (not shown) may be stored within the storage space 2230. Thus, a dry environment can be facilitated within the cartridge device 2000, thereby facilitating storage of the sensor 820.

In some examples, the cartridge device 2000 may also include a platform 2300 (see FIG. 15A). The platform 2300 may be configured to be movable along the mounting table 2200.

In some examples, the platform2300 may include a hollowed-outportion 2310 (see FIG. 15C). In some examples, the hollowed-out profile of the hollowed-out portion 2310 may be substantially the same as the profile of mounting table 2200. In some examples, the platform 2300 is configured to move along the mounting table 2200, and the hollowed-out portion 2310 may be disposed enclosing a periphery of the mounting table 2200 when moving. In this case, when an upper surface of the mounting table 2200 is located below an upper surface or a lower surface of the platform 2300, the sensor 820 mounted on the mounting table 2200 may be hidden inside; the sensor 820 may be exposed as the platform 2300 moves downward and the upper surface of the mounting table 2200 is above the upper surface of the platform 2300.

In some examples, the platform 2300 may include a clamping portion 2320. The clamping portion 2320 may include 2320a and 2320b disposed on opposite sides of the hollowed-out portion 2310. When the sharp object 270 is also packaged within the cartridge device 2000, 2320a and 2320b may clamp the cap portion 276 of the sharp object 270. In this case, the cap portion 276 of the sharp object 270 is clamped by the clamping portion 2320, whereby the sharp object 270 can be more stably packaged in the cartridge device 2000.

In some examples, the platform 2300 may include a supported portion 2350 (see FIG. 15C). By supporting the supported portion2350, the platform 2300 can be positioned above the mounting table 2200. In some examples, the number of supported portion(s) 2350 can be one or more, such as 1, 2, 3, or 4. In the embodiment shown in FIG. 15C, the supported portion 2350 may include 2350a, 2350b, 2350c, and 2350d. In some examples, the plurality of supported portions 2350 may be symmetrically distributed about the periphery of the platform 2300. As a result, it is able to facilitate uniform supporting.

In some examples, the supported portion 2350 may include an arm 2351 protruding outwardly from the main body of the platform 2300 in the direction substantially orthogonal to the axis, a protrusion 2352 protruding further outwardly by the arm 2351 from the main body of the platform 2300 in the direction substantially orthogonal to the axis, and a side plate 2353 protruding laterally from the arm 2351 in a circumferential direction substantially along the platform 2300, and the arm 2351 may have elasticity. In this case, the platform 2300 may be supported through the side plate 2353, and the arm 2351 can be moved toward the main body portion of the platform 2300 by acting on the protrusion 2352, and the side plate 2353 is engaged away from the initial position and the plateform 2300 is no longer supported. When the platform 2300 is no longer supported, the sensor 820 mounted on the mounting table 2200 can be exposed by acting on the platform 2300 toward a bottom portion of the cartridge body 2100, to facilitate subsequent processing, e.g. to facilitate the applicator 1000 to pickup the sensor 820.

In some examples, the platform 2300 may also include a pressed portion 2330 (see FIG. 15C). By pressing the pressed portion 2330, the platform 2300 can be moved toward the bottom portion of the cartridge body 2100 along the mounting table 2200. In some examples, the pressed portion 2330 may protrude outwardly from the body portion of the platform 2300 in the direction substantially orthogonal to the axis. In some examples, the number of the pressed portion(s) 2330 may be one or more, such as 1, 2, 3, or 4. In the embodiment shown in FIG. 15C, the pressed portions 2330 include 2330a, 2330b, 2330c, and 2330d. In some examples, the plurality of pressed portions 2330 may be symmetrically distributed over the periphery of the platform 2300. As a result, it is able to facilitate uniform pressurization

In some examples, the platform 2300 may also include a restricted portion 2360 (see FIG. 15C). The movement of the platform 2300 along the mounting table 2200 can be further defined by applying a restriction to the restricted portion 2360. In some examples, the restricted portion 2360 may be a ridge, or a groove, disposed at the periphery of the platform 2300. In some examples, the number of the restricted portion(s) 2360 may be one or more, such as 1, 2, 3, or 4. In the embodiment shown in FIG. 15C, the restricted portions 2360 are two ridges disposed side by side on the periphery of the platform 2300 in the direction of the axis of the platform 2300 and a groove formed between the two ridges. In addition, in the embodiment shown in FIG. 15C, the restricted portion 2360 may include 2360a, 2360b, and 2360c.

In some examples, the cartridge device 2000 may also include a fence structure 2400 (see FIG. 15A). The fence structure 2400 may be configured to support the platform 2300 and further configured to provide guidance and limitation for movement of the platform 2300.

In some examples, the fence structure 2400 may include a support portion 2410 (see FIG. 15B). Support portion 2410 may support the supported portion 2350 of the platform 2300. In some examples, the support portion 2410 may be a protrusion that protrudes inwardly from a main body portion of fence structure 2400. The number and location of the support portions 2410 corresponds to the number and location of the supported portions 2350 of the platform, e.g. both have the same number, and the projection planes thereof along the axis at least partially coincide. In the embodiment shown in FIG. 15B, the supported portions 2410 may include 2410a, 2410b, 2410c, and 2410d.

In some examples, the fence structure 2400 may also include a restriction portion 2420 (see FIG. 15B). The restriction portion 2420 may define the restricted portion 2360 of the platform 2300. Movement of the platform 2300 can thereby be restricted. In some examples, the restriction portion 2420 may be a ridge-like protrusion protruding inwardly from the main body portion of the fence structure 2400, the ridge-like protrusion may be embedded in a groove of the restricted portion 2360. In the embodiment shown in FIG. 15B, the restriction portions 2420 may be 2420a, 2420b, and 2420c.

FIG. 16A is a schematic diagram showing the mounting table 2200 on which a sensor 820 is mounted according to an example of the present embodiment; FIG. 16B is a schematic diagram showing that the platform 2300 according to an example of the present embodiment is higher than the mounting table 2200; FIG. 16C is a schematic diagram showing that the platform 2300 according to an example of the present embodiment moves toward the bottom portion of the cartridge body 2100 so as to be lower than the mounting table 2200.

In some examples, the fence structure 2400 may also include an avoidance portion 2430 (see FIG. 16A). The avoidance portion 2430 may provide an avoidance space for the pressed portion 2330 when the platform 2300 moves downward. In the embodiment shown in FIG. 16A, the avoidance portions 2430 may include 2430a and 2430b.

With reference to FIGS. 16A, 16B, and 16C, a sharp object 270, a sensor 820, and a connection portion 840 are mounted on the mounting table 2200. When the platform 2300 is in the initial position, the sharp object 270, etc. can be hidden in the hollowed-out portion 2310 of the platform 2300; when the platform 2300 is driven toward the bottom portion of the cartridge body 2100, the mounting table 2200 passes through the hollowed-out portion 2310 and exposes the sharp object 270, etc.

As described above, the supported portion 2350 of the platform 2300 (in particular, the side plate 2353) is supported by the support portion 2410 of the fence structure 2400 so as to be held above the mounting table 2200 so that the sharp object 270 or the like is hidden By acting inwardly on the protrusion 2352, the arm 2351 can pivot inwardly and link the side plate 2353 inwardly away from the support portion 2410 and no longer being supported. In this case, the platform 2300 can be moved downward relative to the mounting table 2200 by applying a force to the platform 2300 toward the bottom portion of the cartridge body 2100.

In some examples, the applicator 1000 may cooperate with the cartridge device 2000 to pickup the sharp object 270, the sensor 820, and the connection portion 840 stored within the cartridge device 2000.

FIG. 17 is a sectional diagram showing the housing 10 according to an example of the present embodiment along the direction substantially orthogonal to the central axis CA.

With reference to FIG. 17, a first type rib 187, a second type rib 188, and a third type rib 189 are provided on the inner wall of the second limiting mechanism 170 of the housing 10.

The first type ribs 187 are configured to cooperate with a guide slot (not shown) in the cartridge device 2000 to assist in picking up in a desired direction. In the embodiment shown in FIG. 17, the first type ribs 187 may include 187a, 187b, 187c, and 187d.

The third type rib 189 may be configured to cooperate with the protrusion 2352 of the supported portion 2350 of the platform 2300, and the second type rib 188 may abut the protrusion 2352 of the supported portion 2350 and actuate the protrusion 2352 inwardly when an action is applied on the applicator 1000 toward the bottom portion of the cartridge body 2100, the arms 2351 pivots inwardly and links the side plates 2353 away from the supported position In some examples, the number of the third type rib(s) 189 may be one or more, such as 1, 2, 3, or 4. In some examples, the number of the third type ribs 189 may be the same as the number of the supported portions 2350 of the platform 2300. In the embodiment shown in FIG. 17, the third type ribs 189 may include 189a, 189b, 189c, and 189d.

The second type ribs 188 are configured to cooperate with the pressed portion 2330 of the platform 2300, and when acting on the applicator 1000 toward the bottom portion of the cartridge body 2100, the second type ribs 188 may abut the pressed portion 2330 and be driven toward the bottom portion of the cartridge body 2100. In some examples, the number of second type rib(s) 188 may be one or more, such as 1, 2, 3, or 4. In some examples, the number of the second type ribs 188 may be the same as the number of the pressing portions 2330 of the platform 2300. In the embodiment shown in FIG. 17, the second type ribs 188 may include 188a, 188b, 188c, and 188d.

FIG. 18A is a schematic diagram showing the applicator 1000 according to an example of the present embodiment before being combined with the cartridge device 2000; FIG. 18b is a schematic diagram showing the applicator 1000 according to an example of the present embodiment after being combined with the cartridge device 2000. The foregoing disclosure describes the structural cooperating relationship of the applicator 1000 with the cartridge device 2000, in which case the application of action to the applicator 1000 toward the cartridge device 2000 will make a pick-up process as shown in FIGS. 18A and 18B happen

FIG. 19A is a schematic diagram showing a case where the sensor 820 is stored in a first exemplary manner by the cartridge device 2000 according to an example of the present embodiment and picked up; FIG. 19B is a schematic diagram showing a case where the sensor820 is stored ina second exemplary manner by the cartridge device 2000 according to an example of the present embodiment and picked up.

In the embodiment shown in FIG. 19A, the sharp object 270, the sensor 820, and the connection portion 840 are stored within the cartridge device 2000 and are picked up by the applicator 1000. In the present embodiment, the sharp object 270 and the holder 280 of the puncture member 260 may be detachable assembly structures. In the picking up process, the cap portion 276 of the sharp object 270 passes through the hole 864 of the attachment portion 860 and the hole 226 of the second bottom portion 220 in turn to be picked up by the sharp object fixing portion 282 and fixed on the holder 280, the connection portion 840 is received and accommodated by the receiving portion 866 of the attachment portion 860, and the attachment portion 860 is received and accommodated by the receiving portion 250. Through this process, the medical instrument 800 is picked up by the applicator 1000. It should be noted that the attachment portion 860 may have been received and accommodated by the applicator 1000 prior to picking.

In the embodiment shown in FIG. 19B, the sensor 820 and the connection portion 840 are stored in the cartridge device 2000, and the sensor 820 and the connection portion 840 are picked up by the applicator 1000. In the present embodiment, the sharp object 270 of the puncture member 260 and the holder 280 may be either detachable or integral. During the picking up procedure, the needle shape portion 272 may in turn pass through the hole 226 of the second bottom portion 220, the hole 864 of the attachment portion 860, and the hole 842 of the connection portion 840, and the implant portion of the sensor 820 is accommodated in the groove 274, the connection portion 840 may be received and accommodated in the receiving portion 866 of the attachment portion 860, and the attachment portion 860 may be received and accommodated in the receiving portion 250. Through this process, the medical instrument 800 is received by the applicator 1000. It should be noted that before picking up, the attachment portion 860 may have been received and accommodated by the applicator 1000, and the needle shape portion 272 may have passed through the hole 226 and the hole 864.

While the invention has been specifically described above with reference to the accompanying drawings and specific embodiments, it is to be understood that the description is not intended to limit the invention in any way. It will be apparent to those skilled in the art that various modifications and variations can be made in the present invention without departing from the substantive spirit or scope of the invention as required, which modifications and variations fall within the scope of the present invention.

## Claims

1. An applicator for applying a medical instrument to a host, **characterized in that**:
the applicator comprises a housing, an auxiliary mechanism, a first drive mechanism and a second drive mechanism, the housing comprising a first holding portion, a proximal end close to the host and a distal end remote from the host in operation, the auxiliary mechanism comprising a moving body releasably held by the first holding portion and configured to be relatively movable to the housing when released, a receiving portion provided to the moving body and configured to receive and accommodate the medical instrument, a second holding portion provided to the moving body, and a puncture member releasably held by the second holding portion and configured to be relatively movable to the receiving portion when released, the first drive mechanism being configured to act on the moving body in a manner toward the proximal end, the second drive mechanism being configured to act on the puncture member in a manner toward the distal end, when released, the moving body being driven by the first drive mechanism toward the proximal end to place the medical instrument received in the receiving portion at least partially subcutaneously in the host through the puncture member, and the puncture member being driven toward the distal end by the second drive mechanism to move the puncture member away from the host.

2. The applicator according to claim 1, **characterized in that**
the moving body comprises a first locking portion configured to releasably interlock with the first holding portion.

3. The applicator according to claim 2, **characterized in that**
the first holding portion and the first locking portion are releasably interlocked by at least one structure of a buckle, a hook, a latch, or a pin.

4. The applicator according to claim 2, **characterized by**
further comprising a first trigger mechanism configured to enable the first holding portion to be separated from the first locking portion for releasing the moving body.

5. The applicator according to claim 1, **characterized in that**
the puncture member comprises a second locking portion configured to releasably interlock with the second holding portion.

6. The applicator according to claim 5, **characterized in that**
the second holding portion and the second locking portion are releasably interlocked by at least one structure of a buckle, a hook, a latch, or a pin.

7. The applicator according to claim 5, **characterized by**
further comprising a second trigger mechanism configured to enable the second holding portion to be separated from the second locking portion for releasing the puncture member.

8. The applicator according to claim 1, **characterized in that**
the housing comprises a first housing having the first holding portion and a second housing mountable to the first housing, the second housing has a first limiting mechanism and a second limiting mechanism communicating with each other, the second limiting mechanism is configured to define an attachment position of the medical instrument, the auxiliary mechanism is mounted to the first limiting mechanism and movable along the first limiting mechanism when the moving body is released by the first holding portion.

9. The applicator according to claim 8, **characterized in that**
the moving body has a first bottom portion close to the distal end, a second bottom portion close to the proximal end, a side wall connecting the first bottom portion and the second bottom portion, and a hollow portion formed between the first bottom portion, the second bottom portion, and the side wall, the receiving portion being provided at the second bottom portion and movable along the hollow portion when the puncture member is released.

10. The applicator according to claim 9, **characterized in that**
the puncture member comprises a sharp object having a groove, and a bearing for supporting the sharp object, the medical instrument may be wholly or partially placed in the groove of the sharp object, and the second drive mechanism is configured to apply an action on the bearing.

11. The applicator according to claim 1, **characterized in that**
the medical instrument comprises a sensor that can be placed subcutaneously in the host, and an attachment portion that is connected to the sensor and can be attached to a body surface of the host.

12. The applicator according to claim 2, **characterized in that**
the first holding portion has a surface facing the distal end, and the first locking portion has a surface facing the proximal end, the surface, facing the distal end of the first holding portion is engaged with the surface, facing the proximal end, of the first locking portion when the moving body is held, and the surface, facing the distal end, of the first holding portion separating from the surface, facing the proximal end, of the first locking portion when the moving body is released.

13. The applicator according to claim 2, **characterized in that**
at least a portion of the first holding portion is "L"-shaped or hook-shaped.

14. The applicator according to claim 2, **characterized in that**
the first holding portion has an arm extending substantially in a direction of a central axis of the applicator, and a protrusion cooperating with the arm and projecting substantially in a direction orthogonal to the central axis of the applicator.

15. The applicator according to claim 14, **characterized in that**
the arm of the first holding portion is resilient in the direction substantially orthogonal to the central axis of the applicator.

16. The applicator according to claim 14, **characterized in that**
the arm of the first holding portion converges toward the central axis of the applicator in a direction from the distal end to the proximal end, and the protrusion of the first holding portion projects toward the central axis of the applicator.

17. The applicator according to claim 14, **characterized in that**
the arm of the first holding portion back-faces away from the central axis of the applicator in a direction from the distal end to the proximal end, and the protrusion of the first holding portion back-faces and projects away from the central axis of the applicator.

18. The applicator according to claim 2 or 16, **characterized in that**
the first locking portion is substantially parallel to or away from a central axis of the applicator in a direction from the proximal end to the distal end.

19. The applicator according to claim 2 or 17, **characterized in that**
the first locking portion is substantially parallel to or converges toward a central axis of the applicator in a direction from the proximal end to the distal end.

20. The applicator according to claim 4, **characterized in that**
the first trigger mechanism has an actuation portion extending substantially in the direction of the central axis of the applicator, and is configured to be movable in the direction of the central axis of the applicator.

21. The applicator according to claim 20, **characterized in that**
When projected in the direction of the central axis of the applicator, a projection of the actuation portion of the first trigger portion at least partially coincides with a projection of the first holding portion, and the first holding portion is actuated to move away from the first locking portion when the first trigger portion moves in the direction of the central axis of the applicator.

22. The applicator according to claim 20, **characterized in that**
When projected in the direction of the central axis of the applicator, a projection of the actuation portion of the first trigger portion at least partially coincides with a projection of the first locking portion, and the first locking portion is actuated to move away from the first holding portion as the first trigger portion moves in the direction of the central axis of the applicator.

23. The applicator according to claim 8, **characterized in that**
the first housing comprises a hollow cylindrical peripheral portion and an end portion provided at one end of the peripheral portion near the distal end, and the first holding portion is provided at the end portion.

24. The applicator according to claim 8, **characterized in that**
one or a plurality of rib shape protrusions are provided on an inner wall of the first housing, and one or a plurality of rib shape grooves are provided on an outer wall of the second housing, and the one or plurality of rib shape protrusions are respectively embedded into the one or plurality of rib shape grooves when the second housing is mounted to the first housing.

25. The applicator according to claim 8, **characterized in that**
the first limiting mechanism and the second limiting mechanism are in hollow cylindrical shape and an inner diameter of the first limiting mechanism is not greater than an inner diameter of the second limiting mechanism.

26. The applicator according to claim 8, **characterized in that**
when the moving body is released, the moving body is movable along the first limiting mechanism and the receiving portion is movable along the second limiting mechanism.

27. The applicator according to claim 8, **characterized in that**
the first limiting mechanism limits a travel of the moving body in a direction of a central axis of the applicator.

28. The applicator according to claim 27, **characterized in that**
When projected in the direction of the central axis of the applicator, a projection of the receiving portion coincides at least partially with a projection of a wall of the first limiting mechanism, and the end, near the distal end, of the moving body coincides at least partially with the wall of the first limiting mechanism.

29. The applicator according to claim 8, **characterized in that**
the first limiting mechanism inhibits rotation of the moving body.

30. The applicator according to claim 29, **characterized in that**
the first limiting mechanism comprises a groove (grooves) and/or a ridge(ridges) provided continuously or discontinuously on an inner wall substantially in a direction of the central axis of the applicator.

31. The applicator according to claim 9, **characterized in that**
a protrusion back-facing away from a central axis of the applicator in a direction generally orthogonal to the central axis of the applicator is provided on an end, near the distal end, of the moving body.

32. The applicator according to claim 9, **characterized in that**
the moving body comprises a groove (grooves) and/or a ridge (ridges) provided on an outer wall of a side wall substantially in a direction of a central axis of the applicator.

33. The applicator according to claim 9, **characterized in that**
the moving body has a cut substantially in a direction of a central axis of the applicator.

34. The applicator according to claim 33, **characterized in that**
a protrusion, protruding toward the central axis of the applicator via the cut, is provided on an inner wall of the first limiting mechanism.

35. The applicator according to claim 9, **characterized in that**
the moving body is configured to inhibit a rotation of the puncture member.

36. The applicator according to claim 35, **characterized in that**
the moving body comprises a groove (grooves) and/or a ridge(ridges) provided on an inner wall of a side wall substantially in a direction of a central axis of the applicator.

37. The applicator according to claim 10, **characterized in that**
the sharp object is integrally formed with the bearing.

38. The applicator according to claim 10, **characterized in that**
the sharp object is configured for removable engagement with the bearing.

39. The applicator according to claim 10, **characterized in that**
the sharp object has a needle shape portion and a cover portion in fixed connection with the needle shape portion, and the bearing has a plurality of finger shape portions converging toward one another, and the cover portion is releasably clamped by the plurality of finger shape portions.

40. The applicator according to claim 10, **characterized in that**
the puncture member comprises a second locking portion configured to releasably interlock with the second holding portion and provided on the bearing, the second locking portion comprises an arm extending substantially in a direction of a central axis of the applicator, and a protrusion cooperating with the arm and back-facing away from the central axis of the applicator substantially in a direction orthogonal to the central axis of the applicator.

41. The applicator according to claim 40, **characterized in that**
the arm of the second locking portion is resilient in the direction substantially orthogonal to the central axis of the applicator.

42. The applicator according to claim 40, **characterized in that**
the moving body has a cut substantially in the direction of the central axis of the applicator, and the protrusion of the second locking portion passes through the cut when the puncture member is held.

43. The applicator according to claim 40, **characterized in that**
when the puncture member is released, the arm of the second locking portion is pressed toward the central axis of the applicator.

44. The applicator according to claim 10, **characterized in that**
the bearing comprises a groove (grooves) and/or a ridge (ridges) provided on an outer wall substantially in the direction of the central axis of the applicator.

45. The applicator according to claim 10, **characterized in that**
the bearing of the puncture member is provided in the hollow portion, the receiving portion is provided at the second bottom portion, the second bottom portion has a through hole, and the sharp object passes through the through hole of the second bottom portion.

46. The applicator according to claim 10, **characterized in that**
when the puncture member is held, a distance between the bearing and the first bottom portion is not less than a length of the sharp object protruding from the receiving portion.

47. The applicator according to claim 5, **characterized in that**
the second holding portion is releasably interlocked with the second locking portion by at least one of a buckle, a hook, a latch, or a pin when the puncture member is held.

48. The applicator according to claim 10, **characterized in that**
the second drive mechanism is disposed between the bearing and the second bottom portion.

49. The applicator according to claim 1, **characterized in that**
the second drive mechanism is a spring.

50. The applicator according to claim 1, **characterized in that**
the first drive mechanism is a spring.

51. The applicator according to claim 11, **characterized in that**
the sensor is configured to react with an analyte in body fluid and generate analyte information.

52. The applicator according to claim 51, **characterized in that**
the analyte is one or more than one of : acetylcholine, amylase, bilirubin, cholesterol, chorionic gonadotropin, creatine kinase, creatine, creatinine, DNA, fructosamine, glucose, glutamine, growth hormone, hormone, ketone body, lactate, oxygen, peroxide, prostate specific antigen, prothrombin, RNA, thyroid stimulating hormone, or troponin.

53. The applicator according to claim 11, **characterized in that**
the sensor comprises an implant portion which can be positioned subcutaneously in the host, and a connection portion connecting the implant portion to the attachment portion.

54. The applicator according to claim 11, **characterized in that**
the sensor is detachably mounted to the attachment portion.

55. The applicator according to claim 11, **characterized in that**
the attachment portion is configured to supply power to the sensor and receive information generated by the sensor.

56. The applicator according to claim 11, **characterized in that**
the attachment portion has a hole penetrating from an upper surface to a lower surface, and an axis of the sensor passes through the hole when the sensor is mounted to the attachment portion.

57. The applicator according to claim 11, **characterized in that**
the attachment portion is configured to adhere to a body surface of the host.

58. The applicator according to claim 11, **characterized in that**
the attachment portion has an initial state configured to be an open circuit and an operation state configured to be a closed circuit.

59. The applicator according to claim 11, **characterized in that**
the attachment portion is configured to communicate with an external device through wireless communication or wired communication.

60. The applicator according to claim 11, **characterized in that**
the attachment portion is configured to be excited from the initial state to the operation state by magnetic action or light action.
